# EUROPEAN PATENT APPLICATION

(11) **EP 4 741 416 A1**
(43) Date of publication of application: **13.05.2026**
(21) Application number: 24212082.2
(22) Date of filing: 11.11.2024
(51) Int. Cl.: C07K 14/32, C12N 9/58

(54) **METHOD FOR PRODUCING A TRYPSIN-LIKE PROTEASE IN BACILLUS**

(71) Applicant: Kerry Group Services International Ltd, Tralee, Co. Kerry, V92 EH11 (IE)
(72) Inventor: RZEZNICKA, Kamila, 04105 Leipzig (DE); HOFFMANN, Gregor, 04416 Markkleeberg (DE)
(74) Representative: Bird & Bird Società tra Avvocati S.r.l.

(57) **Abstract**

The invention relates to a method for producing a trypsin-like protease in *Bacillus,* the method comprising expressing in a *Bacillus* host cell a gene coding for a preproenzyme comprising or consisting of (i) a trypsin-like protease having a sequence identity of at least 70% compared to the wild-type trypsin-like protease from the fungus *Fusarium oxysporum,* and (ii) preferably, a prosequence located upstream of the trypsin-like protease and having at its C-terminus a basic amino acid residue, and (iii) a bacterial secretion signal sequence located upstream of the trypsin-like protease and of the optionally present prosequence, wherein the bacterial secretion signal sequence is capable of directing secretion in a *Bacillus* host cell to a culture medium, thereby providing a culture medium comprising the mature trypsin-like protease.

## Description

The invention relates to a method for producing a trypsin-like protease in *Bacillus,* the method comprising the steps of (a) expressing in a *Bacillus* host cell a gene coding for a preproenzyme comprising or consisting of a trypsin-like protease having a sequence identity of at least 70% compared to SEQ ID NO:1, which is the wild-type trypsin-like protease from the fungus *Fusarium oxysporum,* and preferably, a prosequence located upstream of the trypsin-like protease and having at its C-terminus a basic amino acid residue, more preferably K or R, and a bacterial secretion signal sequence located upstream of the trypsin-like protease and of the optionally present prosequence, wherein the bacterial secretion signal sequence is capable of directing secretion in a *Bacillus* host cell to a culture medium, preferably wherein the bacterial secretion signal sequence originates from *Bacillus,* thereby providing a culture medium comprising the mature trypsin-like protease. Optional steps may include (b) working-up the cell culture obtained in step (a) thereby providing a solid cell culture residue and a liquid cell culture supernatant, optionally (c) purifying the mature trypsin-like protease from the cell culture supernatant, optionally (d) formulating the mature trypsin-like protease, and optionally (e) packaging the purified mature trypsin-like protease. The invention further relates to a preproenzyme, preferably as defined according to any embodiment of the first aspect of the invention. The invention also relates to an expression vector comprising a DNA sequence coding for the preproenzyme according to the second aspect of the invention. Moreover, the invention relates to a host cell comprising a DNA sequence coding for the preproenzyme according to the second aspect of the invention or the third aspect of the invention.

Trypsin (EC 3.4.21.4) is a serine protease with stringent cleavage specificity for the carboxyl termini of arginine and lysine and is integral to numerous biological functions, e.g., proteolysis in digestion, signal transduction and immune response. In its natural form, trypsin is synthesized as an inactive precursor, the preproenzyme, having an N-terminal secretion signal peptide, followed by a short prosequence. The secretion signal peptide is removed upon extracellular trafficking and activation of the proenzyme (trypsinogen), the second protease precursor, is achieved by proteolytic removal of the prosequence, catalyzed e.g., by a second enzyme like an enterokinase or by trypsin through autoactivation. Trypsin or trypsin-like proteases are found in various different organisms, including mammals, birds, fishes, amphibians, primitive vertebrates, insects, fungi, and bacteria. Up to date, trypsin has been utilized in many industrial applications, e.g., for food processing, leather bating, detergents, or pharmaceutical applications like the manufacturing of insulin, vaccines or viral vectors. The commercial production of trypsin usually involves animal sources from bovine or porcine origin. However, the usage of animal-derived trypsin harbors the potential risk of contamination with infectious agents like viruses or other pathogens, which cannot be neglected when used in the production of medicinal products like vaccines or viral vectors. Recombinant trypsin eliminates this risk, making it a safer alternative for use in biopharmaceutical manufacturing. Furthermore, the activity and quality of recombinant trypsin can be more consistent as it is not subject to the same biological variability as animal-derived trypsin. The use of recombinant trypsin additionally avoids ethical issues associated with the use of animal products. Further benefits of some forms of recombinant trypsin are advantages in room-temperature stability, an enhanced performance, or avoiding the need of protease inhibitors. In summary, recombinant trypsin offers a safer, more consistent, and potentially more efficient alternative to animal-derived forms, why it is gradually being adopted in various industrial processes.

The trypsin of the fungus *Fusarium oxysporum* (FOT) is a serine protease with similar characteristics to mammalian trypsin and thus appears to be a suitable target for the recombinant production of a non-animal derived trypsin variant. However, it has a distinct prosequence, which does not enable autoactivation and is naturally hydrolyzed by a specific extracellular *Fusarium* aspartyl protease. Recombinant expression of FOT has been shown in different expression hosts, e.g., in *Aspergillus oryzae, Pichia pastoris* and *Escherichia coli.* However, when expressed in a non-*Fusarium* host, low yields and difficulties with trypsin-activation have been reported. Therefore, other expression hosts have to be considered to provide an industrially advantageous production of trypsin. An increasingly preferred host for the expression of recombinant proteins with medical, biotechnological, and industrial value is *Bacillus,* e.g., *Bacillus amyloliquefaciens* and *Bacillus subtilis,* as it is generally recognized as safe (GRAS status by FDA), provides high fermentation yields and is additionally characterized by the absence of exotoxins and endotoxins. According to our knowledge, no expression of FOT in *Bacillus* has been reported so far.

WO1994025583A1 refers to a recombinant expression of FOT in *Aspergillus oryzae.* The trypsin from *Fusarium oxysporum* is expressed in an inactive form and is activated by the addition of an aspartyl protease.

WO2018174231A1 refers to the expression of *Fusarium oxysporum* derived trypsin-like protease variants in yeast cells *(Pichia pastoris).* Autodigestion/autoactivation occurred for some variants but only after rebuffering the culture supernatant and following an additional 24h to 48h incubation. Furthermore, fairly low protease activities could be measured (below 0.6 kU/1). Autoactivation was not observed with the *Fusarium oxysporum* wild-type prosequence.

CN115216463A refers to the recombinant expression of FOT in E. *coli,* reporting the exchange of the prosequence to an enterokinase cleavage site, which requires the addition of enterokinase for trypsin activation.

However, the FOT expression systems of prior art are either not satisfactory in trypsin yields, or do not result in autocatalytic activation of the FOT proenzyme to trypsin. To achieve activation, either the addition of a second protease is required or rebuffering of the cell culture supernatant is necessary, all leading to complex and cost intensive processes. Further, recombinant FOT expression described in prior art does not mention the usage of *Bacillus* hosts, which are favorable expression hosts for a variety of applications. Hence, there still remains a demand for improved methods or systems for the production of *Fusarium oxysporum* trypsin-like protease, which enables an improved activation mechanism of the preproenzyme and consequently improved yields, leading to efficient and cost optimized processes.

It is an object of the invention to provide a method for the production of a trypsin-like protease that has advantages compared to the expression methods of the prior art. In particular, it is an object of the invention to provide a method for the production of a trypsin-like protease in a host organism that is able to obtain the mature trypsin-like protease in the culture medium from the preproenzyme without adding other proteases to cleave the prosequence from the trypsin-like protease, preferably due to autoactivation without the need of rebuffering steps. Another object of the invention is to provide a method for the production of a trypsin-like protease in a host organism that is useful to obtain superior volumetric activities (or volumetric activities recoverable) of the mature trypsin-like protease in the culture medium and consequently superior yields of the mature trypsin-like protease.

This object has been achieved by the subject matter of the patent claims.

In summary, the invention provides a method for producing a trypsin-like protease in *Bacillus,* essentially comprising expressing in a *Bacillus* host cell a gene coding for a preproenzyme comprising or consisting of (i) a trypsin-like protease having a sequence identity of at least 70% compared to SEQ ID NO:1 *(Fusarium oxysporum* trypsin-like protease) and (ii) a bacterial secretion signal sequence located upstream of the trypsin-like protease, preferably wherein the bacterial secretion signal sequence originates from *Bacillus,* thereby providing a culture medium comprising the trypsin-like protease.

It has been surprisingly found and demonstrated in the experimental section that by means of the method according to the invention, significantly higher volumetric activities of the trypsin-like protease can be obtained in the culture medium when the prosequence is absent, compared to the method comprising the prosequence consisting of SEQ ID NO:2, which is the wild-type prosequence of *Fusarium oxysporum* trypsin-like protease.

Further, it has been surprisingly found that when the preproenzyme is expressed by the method according to the invention, the trypsin-like protease preproenzyme comprising the prosequence consisting of SEQ ID NO:2 (wild-type prosequence of *Fusarium oxysporum* trypsin-like protease) is activated in the culture medium, leading to volumetric activities (recoverable) of more than 2 kU/l as demonstrated in the experimental section. Additionally, the activation of the trypsin-like protease does not require the supplementation of an external protease or rebuffering steps.

The invention further relates to a method for producing a trypsin-like protease in *Bacillus,* essentially comprising expressing in a *Bacillus* host cell a gene coding for a preproenzyme comprising or consisting of (i) a trypsin-like protease having a sequence identity of at least 70% compared to SEQ ID NO: 1 *(Fusarium oxysporum* trypsin-like protease), (ii) a prosequence located upstream of the trypsin-like protease, containing at its C-terminus a basic amino acid residue, preferably K or R, more preferably wherein the prosequence consists of an amino acid sequence having a sequence identity of at least 70% compared to any of SEQ ID NO:7 to SEQ ID NO:37, most preferably compared to any of SEQ ID NO:7 to SEQ ID NO:26. and (iii) a bacterial secretion signal sequence located upstream of the trypsin-like protease, preferably wherein the bacterial secretion signal sequence originates from *Bacillus,* thereby providing a culture medium comprising the mature trypsin-like protease.

It has been surprisingly found that when the method is realized according to the invention comprising prosequences having at its C-terminus a basic amino acid residue, preferably K or R, significantly higher volumetric activities can be obtained in the culture medium compared to the method comprising the prosequence consisting of SEQ ID NO:2 (wild-type prosequence of *Fusarium oxysporum* trypsin-like protease). Surprisingly, particularly preferred prosequences having sequence identities of at least 70% compared to any of SEQ ID NO:7 to SEQ ID NO:37 and more preferably compared to any of SEQ ID NO:7 to SEQ ID NO:26 resulted in even more improved volumetric activities of the mature trypsin-like protease in the culture medium as demonstrated in the experimental section.

A first aspect of the invention relates to a method for producing a trypsin-like protease in *Bacillus,* the method comprising the steps of
(a) expressing in a *Bacillus* host cell a gene coding for a preproenzyme comprising or consisting of
   - a trypsin-like protease having a sequence identity of at least 70% compared to SEQ ID NO: 1;
   - preferably, a prosequence located upstream of the trypsin-like protease and having at its C-terminus a basic amino acid residue, more preferably K or R; and
   - a bacterial secretion signal sequence located upstream of the trypsin-like protease and of the optionally present prosequence, wherein the bacterial secretion signal sequence is capable of directing secretion in a *Bacillus* host cell to a culture medium, preferably wherein the bacterial secretion signal sequence originates from *Bacillus;*
   thereby providing a culture medium comprising the mature trypsin-like protease;
(b) optionally, working-up the cell culture obtained in step (a) thereby providing a solid cell culture residue and a liquid cell culture supernatant;
(c) optionally, purifying the mature trypsin-like protease from the cell culture supernatant;
(d) optionally, formulating the mature trypsin-like protease; and
(e) optionally, packaging the purified mature trypsin-like protease.

A particularly preferred embodiment of the invention relates to a method for producing a trypsin-like protease in Bacillus, the method comprising the steps of
(a) expressing in a Bacillus host cell a gene coding for a preproenzyme comprising or consisting of
   - a trypsin-like protease having a sequence identity of at least 70% compared to SEQ ID NO: 1;
   - a prosequence located upstream of the trypsin-like protease and having at its C-terminus a basic amino acid residue, preferably K or R, more preferably wherein the prosequence consists of an amino acid sequence having a sequence identity of at least 70% compared to any of SEQ ID NO:7 to SEQ ID NO:37, preferably compared to any of SEQ ID NO:7 to SEQ ID NO:26.; and
   - a bacterial secretion signal sequence located upstream of the trypsin-like protease and of the prosequence, wherein the bacterial secretion signal sequence is capable of directing secretion in a *Bacillus* host cell to a culture medium, preferably wherein the bacterial secretion signal sequence originates from *Bacillus;*
   thereby providing a culture medium comprising the mature trypsin-like protease;
(b) optionally, working-up the cell culture obtained in step (a) thereby providing a solid cell culture residue and a liquid cell culture supernatant;
(c) optionally, purifying the mature trypsin-like protease from the cell culture supernatant;
(d) optionally, formulating the mature trypsin-like protease; and
(e) optionally, packaging the purified mature trypsin-like protease.

For the purpose of the specification, a *trypsin-like protease* (EC 3.4.21.4) is a serine endopeptidase with trypsin-like substrate specificity. Serine proteases are enzymes that can cleave peptide bonds and trypsin-like substrate specificity means that such enzymes cleave peptide bonds specifically at the carboxyl side of a positively charged amino acid, preferably of lysine or arginine. Trypsin-like proteases are expressed as *preproenzymes,* which are inactive precursors of the mature trypsin-like proteases. The *preproenzyme* comprises the sequence of the mature trypsin-like protease, a prosequence which is located upstream (N-terminal) of the trypsin-like protease sequence and further comprises a secretion signal sequence, which is located upstream (N-terminal) of the prosequence. The secretion signal sequence is responsible for the extracellular secretion and folding of the trypsin-like protease and its amino acid sequence is cleaved off from the overall amino acid sequence during the secretory process, resulting in the formation of another form of the inactive trypsin-like protease precursor, the *proenzyme,* which is secreted in the culture medium. It comprises the prosequence and the amino acid sequence of the trypsin-like protease without the N-terminal secretion signal sequence. The proenzyme of the trypsin-like protease is finally activated in the culture medium after the cleavage of the prosequence moiety, resulting in the release of the mature trypsin-like protease. For the purpose of the specification, *mature* trypsin-like protease shall mean the activated form after the maturation process from the preproenzyme to the proenzyme and to the active trypsin-like protease which includes the cleavage of the bacterial secretion signal sequence and the prosequence from the trypsin-like protease moiety.

The amino acid sequence of SEQ ID NO: 1 corresponds to the sequence of the mature trypsin-like protease from the fungus *Fusarium oxysporum.*

For the purpose of the specification, the term *upstream* shall refer to a relative position of amino acid sub-sequences, which are located N-terminal to a reference amino acid sequence or amino acid residues. The term *downstream* refers to amino acid sub-sequences that are located C-terminal of a reference amino acid sequence or amino acid residues.

For the purpose of the specification, the *prosequence* of the trypsin-like protease is a N-terminal short amino acid sequence. During the activation process of the trypsin-like protease, the prosequence is hydrolyzed from the proenzyme (or precursor) by a physical activator which can be the trypsin-like protease itself or another protease. In one embodiment, the prosequence is optional for the production of the trypsin-like protease in *Bacillus.* In preferred embodiments of the method according to the invention, the prosequence is comprised in the preproenzyme.

For the purpose of the specification, the *bacterial secretion signal sequence* is a short amino acid sequence that can direct proteins into the secretory pathway to the extracellular matrix in bacterial expression hosts, such as *Bacillus.* The secretion of proteins is a universally conserved process with critical functions in cell physiology and industrial applications. Secretory proteins are synthesized initially as pre-proteins with an N-terminal signal peptide extension, which distinguishes the extracellular exported proteins from the cytoplasmatic ones. The length of bacterial secretion signal sequences varies usually between 15 and 35 amino acid residues.

For the purpose of the specification, a bacterial secretion signal sequence *"capable of directing secretion in a Bacillus host cell to a culture medium"* refers to any signal peptide that is capable to traffic a polypeptide to the extracellular matrix via the secretory pathway in the expression host belonging to the genus *Bacillus.* Such a secretion signal sequence is not particularly limited to originate from *Bacillus* and might be derived from an organism other than *Bacillus.* Numerous secretion signal sequences functioning in *Bacillus* are known and examples of such signal sequences are specified in the following literature: Tjalsa et.al. DOI: 10.1128/mmbr.64.3.515-547.2000 and DOI: 10.1128/MMBR.68.2.207-233.2004. They share certain typical structural features and generally contain three distinct domains: N, H and C. The N-terminal located N-domain contains at least one positively charged residue e.g., arginine or lysine, being responsible for the interaction with the negatively charged phospholipids in the membrane. The H-region, which follows downstream the N-region, is formed by a stretch of hydrophobic residues that can insert into the hydrophobic membrane. These are followed downstream by the C-domain that contains the cleavage site for specific signal peptide proteases, the so called SPases, which remove the signal peptide from the target protein during the secretion process. Typically, such C-domains comprise the consensus sequence A-X-A at position - 3 to -1 amino acids of the SPase cleavage site, wherein A refers to an alanine residue and X refers to any amino acid residue.

For the purpose of the specification, the percent identity is calculated as: Sequence Identity [%] = number of Matches/ L x 100, wherein L is the number of aligned positions, i.e. identities and non-identities (including gaps, if any). Identity is preferably calculated using BLASTP (see, for example, Altschul SF et al. (1997) "Gapped BLAST and PSI-BLAST: a new generation of protein database search programs", Nucleic Acids Res. 25:3389-3402; or Altschul SF (2005) "Protein database searches using compositionally adjusted substitution matrices." FEBS J. 272:5101-5109); preferably with the following algorithm parameters: Matrix: BLOSUM62; Gap Costs: Existence: 11 Extension: 1, Expect threshold: 0.05, and Word size: 6. BlastP can be accessed online at the NCBI Homepage (https:// blast.ncbi.nlm. nih.gov/Blast.cgi?PROGRAM=blastp&PAGE_TYPE=BlastSearch&LINK_LOC=blasthome). Other program settings can be adjusted as desired, for example using the following settings:
- Field "Enter Query Sequence": Query subrange: none;
- Field "Choose Search Set": Database: non-redundant protein sequences (nr); optional parameters: none
- Field "Program Selection": Algorithm: blastp (protein-protein BLAST);
- Algorithm parameters: Field "General parameters": Max target sequences: 5000; Short queries: Automatically adjust parameters for short input sequences; Expect threshold: 0.05; Word size: 6; Max matches in a query range: 0;
- Algorithm parameters: Field "Scoring parameters": Matrix: BLOSUM62; Gap Costs: Existence: 11 Extension: 1; Compositional adjustments: Conditional compositional score matrix adjustment;
- Algorithm parameters: Field "Filters and Masking": Filter: none; Mask: none.

In addition to the default parameters for calculation of percent identity, when aligning a reference sequence, referred to as *"query sequence",* with another sequence, referred to as *"subject sequence",* in particular wherein the query sequence is any of the sequences provided by the present invention; preferably SEQ ID NO: 1 and any of SEQ ID NO:7 to SEQ ID NO:37, and wherein the subject sequence is any other sequence, for example a sequence disclosed in a database, the subject sequence must align over a sequence stretch covering at least 70% of the overall length of the query sequence *("sequence coverage"* of at least 70%); the alignment of both sequences must cover at least 70% of the query sequence; for the purpose of specification, the sequence coverage is calculated as: the number of aligned positions, i.e. covering identities and non-identities (including gaps, if any) divided by the overall length of the query sequence and multiplied by 100. Alignments with a lower sequence coverage of the query sequence by the subject sequence are excluded for the determination of sequence identity for the purposes of this application. However, within the sequence coverage, the subject sequence may be longer or shorter than the length of the alignment.

In the context of the specification, the natural proteinogenic amino acids can be divided into different classes according to their physicochemical properties as specified by their side chains. The classes can be thereby defined on the hydropathy, volume, chemical nature, charge, and polarity (Pommié, C. et al., J. Mol. Recognit., 17, 17-32 (2004)). Preferably, for the purpose of the specification there are seven classes of amino acid residues, which are defined based on the chemical structure (i) aliphatic amino acids: A, G, I, L, P, V, (ii) aromatic amino acids: F, W, Y, H, (iii) sulfur amino acids: C, M, (iv) hydroxyl amino acids S, T, (v) basic amino acids: R, H, K, (vi) acidic amino acids D, E, (vii) amide amino acids N, Q.

For the purpose of the specification, *"culture medium"* refers to a solution or liquid formulation medium in which host cells are cultivated. It is designed to support the growth and maintenance of a population of microorganisms or cells, e.g., *Bacillus* host cells for recombinant protein expression. The culture medium provides essential nutrients, including carbon sources (e.g., glucose, glycerol), nitrogen sources (e.g., ammonium salts, amino acids), and minerals (e.g., magnesium, potassium), along with buffering agents to maintain a stable pH, typically between 6 and 8. It may also include vitamins, cofactors, or trace elements to enhance cell growth and productivity. The host cells are cultivated within the culture medium, with adjustments to pH, temperature, or nutrient concentration as needed to optimize the expression and secretion of the specific target, such as the trypsin-like protease, ensuring the stability, yield, and activity of the enzyme in the extracellular environment.

In the context of the specification, "*working-up the cell culture"* refers to the process of treating or processing a cell culture after fermentation or cultivation to separate and recover its components. This process involves but is not limited to using techniques such as filtration, centrifugation, or other suitable separation methods to divide the culture into two main fractions: a solid cell culture residue, which consists of non-soluble components such as cells, cell debris, or other particulate matter derived from the host cells, and a liquid cell culture supernatant, which is the clarified cell-free liquid portion containing soluble components like secreted proteins (like the proenzyme or the mature trypsin-like protease), and other molecules that have been released into the culture medium during the fermentation or cultivation process. The result of working-up the cell culture is the separation of these distinct fractions for further downstream processing or analysis.

In the context of the specification, the term *"purifying the trypsin-like protease from the cell culture supernatant"* refers to the process of isolating and concentrating the trypsin-like protease from the liquid fraction of the culture medium after it has been separated from the solid cell culture residue. This purification process typically involves a series of steps designed to remove impurities, such as other proteins, metabolites, and residual cell components, while retaining the mature (active) trypsin-like protease. Common techniques for purification may include, but are not limited to, filtration, precipitation, chromatography (e.g., affinity, ion exchange, or size-exclusion), and dialysis. The goal of purifying the trypsin-like protease is to obtain it in a form that is substantially free from contaminants, with high specificity and activity.

In the context of the specification, the term "*formulating the mature trypsin-like protease"* refers to the process of preparing the purified, mature (active) trypsin-like protease into a specific composition or mixture suitable for its intended use. This process typically involves combining the protease with excipients, stabilizers, preservatives, or other additives to ensure its stability, activity, and compatibility with the desired application. Formulation may include adjusting factors such as pH, concentration, and the medium in which the protease is suspended or dissolved to optimize its performance and shelf-life. As part of this process, sterile filtration may be employed to further remove any residual particulates or microbial contaminants, ensuring that the formulation remains sterile. Sterile filtration, along with other formulation steps such as adjusting pH, concentration, and medium composition, ensures that the final product is stable, active, and free from contaminants, making it suitable for use in industrial, pharmaceutical, or biochemical applications while maintaining the required sterility and quality.

In the context of the specification, the term *"packaging the purified mature trypsin-like protease "refers* to the process of enclosing or containing the purified and formulated protease in suitable containers or vessels under sterile conditions to ensure the product's integrity, stability, and safety. This packaging process ensures the protease is protected from environmental factors, such as moisture, light, or contamination that could affect its stability and activity. The packaging may involve sealing the protease in appropriate materials, such as vials, bottles, ampoules, or bulk containers, depending on the intended use or application. The use of sterile conditions during packaging is crucial to prevent microbial contamination and to maintain the protease's purity and effectiveness throughout storage, transport, and distribution.

In preferred embodiments of the method according to the invention, the prosequence consists of an amino acid sequence having a sequence identity of at least 70% compared to any of SEQ ID NO:7 to SEQ ID NO:37, preferably compared to any of SEQ ID NO:7 to SEQ ID NO:26.

In preferred embodiments of the method according to the invention, the prosequence has at its N-terminus an aliphatic amino acid residue, preferably A.

In preferred embodiments of the method according to the invention, the prosequence consists of 10 amino acid residues.

In preferred embodiments of the method according to the invention, the prosequence consists of 9 amino acid residues.

In preferred embodiments of the method according to the invention, the prosequence consists of 8 amino acid residues.

In preferred embodiments of the method according to the invention, the prosequence consists of 7 amino acid residues.

In preferred embodiments of the method according to the invention, the prosequence consists of 6 amino acid residues.

In preferred embodiments of the method according to the invention, the prosequence consists of 5 amino acid residues.

In preferred embodiments of the method according to the invention, the prosequence consists of 4 amino acid residues.

In preferred embodiments of the method according to the invention, the prosequence consists of 3 amino acid residues.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:7.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:8.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:9.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:10.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:11.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:12.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:13.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:14.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 15.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 16.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 17.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 18.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 19.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:20.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:21.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to the amino acid sequence APR (also referred to as *"SEQ ID NO:22").*

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:23.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:24.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:25.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:26.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:27.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:28.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:29.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:30.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:31.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:32.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:33.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:34.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:35.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:36.

In preferred embodiments of the method according to the invention, the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:37.

In preferred embodiments of the method according to the invention, the bacterial secretion signal is directly linked to the prosequence.

In preferred embodiments of the method according to the invention, the prosequence is directly linked to the trypsin-like protease.

In preferred embodiments of the method according to the invention, the bacterial secretion signal sequence originates from a species within the genus *Bacillus* selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis.*

In preferred embodiments of the method according to the invention, the bacterial secretion signal sequence is the secretion signal sequence from secretory *Bacillus* proteins selected from the group consisting of YjfA, YfhK, Csn, LytD, Bpr, WapA, BglC/EglS, LytB, LipA/EstA, YckD, YbdN, YobB, YhfM, BglS, YjdB, YbbE, GlpQ, SacC, YurI/Bsn, PhoB, YdjM, AbnA, YwjE/ClsB, YqgA, LipB/EstB, FliZ, DacB, SacB, YrvJ, YlaE, YqxI, NprB, YbfO, YlqB, SpoIID, YwmC, YvbX, YkvV/StoA, XynA, YbbC, YkvT, NprE, YolC, YqzG, YvcE/CwlO, YkwD, YqxM, Mpr, TasA, YwmD, DacF, LytF, YjiA, PbpD, PelB, SpoIIQ, CccA, CitH/mdh, AspB, YlbL, YoqH, YpbG, YpcP/ExoA, YpuD, YvpB, YwcI, YweA, YwgB, YwmB, YwoF, YwsB, YwtF, Pel, PenP, LytC, YfkN, YngK, YwqC, RpmG, YojL/CwlS, YncM, PhrK, Vpr, PhrC, YfkD, YolA, YoaW, YlxY, SleB, YxiT, NucB, YpuA, YwaD, YpjP, WprA, YjcM, MotB, YdhT/GmuG, LytE, PhrF, YlxW, YobV, YocH, YodV/Phy, YoqM, YraJ, YuaB, YusW, YvgO, YvnB, YxiA/AbnB, CwlD, DltD, FliL, LytR, MreC, PbpB, PhoA, SpoIIP, SpoIIR, YrrR/PbpI, YrrS, YveB/LevB, LytH, YbdG, PbpX, YddT, YjcN, YolIBdbA, YqzC, YlxF, YndA, YkoJ, YwtD/PgdS, YdbK, YbbR, YknX, YnzA/TatAC, YpmS, YvgV/BdbD, AmyE, YybN, YwfM, YhdC, YxaK/YxaL, YopL, YhjA, PhrG, YvpA/PelC and PhrA; preferably PhoA, YncM, YqgA, YjfA, YfkD, StoA, YdjM, DacF, Vpr, YbfO, AbnA, LytC, PgdS, YckD, YpuA, YuaB, YvnB, YxaL, YoqH and YbdN.

For the purpose of clarification, protein names of secretory *Bacillus* proteins are presented in accordance with standard conventions used in scientific databases such as NCBI and UniProt. These databases often list genes and the amino acid sequences they encode using the same name. However, to distinguish between gene and protein names, gene names are typically written in lowercase, while protein names are written with an initial capital letter. For instance, a gene referred to as "*yjfA*" would correspond to a protein named *"YjfA".*

For the purpose of clarification, a person skilled in the art can retrieve amino acid sequences of secretory proteins like those individualized above from databases like "NCBI - GenBank" or "UniProt - UniProtKB" for any species, preferably for the species as individualized above by typing the name of the protein and the name of the species into the query form.

For the purpose of the specification, a person skilled in the art can extract signal peptide amino acid sequences from amino acid data of secretory proteins like those individualized or even from proteome data because the signal peptides are either already known or assigned in database entries, e.g. "NCBI - GenBank" or "UniProt - UniProtKB", for the corresponding secretory proteins. When using the database "UniProt", the person skilled in the art can directly retrieve the amino acid sequence corresponding to the secretion signal sequence of the respective protein in the "Features" section of the data base entry. Alternatively, signal peptide amino acid sequences can be predicted from amino acid sequence data as they are characterized by distinct classes that are known by the skilled person. According to Teufel et.al. DOI: 10.1038/s41587-021-01156-3, such classes include but are not limited to (i) Sec/SPI ("standard" secretory signal peptides transported by the Sec pathway and cleaved by Signal Peptidase I)-, (ii) Sec/SPII- (lipoprotein signal peptides transported by the Sec pathway and cleaved by Signal Peptidase II), (iii) Tat/SPI- (Tat signal peptides transported by the Tat pathway and cleaved by Signal Peptidase I, (iv) Tat/SPII- (Tat lipoprotein signal peptides transported by the Tat pathway and cleaved by Signal Peptidase II) and (v) Sec/SPIII- (Pilin and pilin-like signal peptides transported by the Sec pathway and cleaved by Signal Peptidase III) signal peptides, which can be distinguished according to characteristic sequence properties/regions. Various bioinformatic tools are available to predict signal peptide sequences according to such properties and some of them use machine learning models to enhance the prediction accuracy.

In preferred embodiments of the method according to the invention, step (a) involves bioinformatically determining the bacterial secretion signal sequence, preferably using bioinformatic tools selected from the group consisting of SignalP, Phobius, PrediSi, SIGCLEAVE, ANTHEPROT - Signal prediction, SOSUIsignal, Signal Find Server, SPD - Secreted Protein Database and SPEPlip.

In preferred embodiments of the method according to the invention, the bacterial secretion signal sequence consists of an amino acid sequence having a sequence identity of at least 70% compared to any of SEQ ID NO:39 to SEQ ID NO:206, preferably to any of SEQ ID NO:39 to SEQ ID NO:75, more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52, SEQ ID NO:56, SEQ ID NO:58, SEQ ID NO:61, SEQ ID NO:62, SEQ ID NO:63, SEQ ID NO:64, SEQ ID NO:66, SEQ ID NO:68, SEQ ID NO:68, SEQ ID NO:70 and SEQ ID NO:74, yet more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52 and SEQ ID NO:56.

Preferably, the sequence identity is at least 73%, preferably at least 75%, more preferably at least 77%, still more preferably at least 80%, yet more preferably at least 83%, even more preferably at least 85%, most preferably at least 87%, and in particular at least 90%, or at least 91%, preferably at least 92%, more preferably at least 93%, still more preferably at least 94%, yet more preferably at least 95%, even more preferably at least 96%, even more preferably at least 97%, even more preferably at least 98%, most preferably at least 99%, in particular100% compared to any of SEQ ID NO:39 to SEQ ID NO:206, preferably to any of SEQ ID NO:39 to SEQ ID NO:75, more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52, SEQ ID NO:56, SEQ ID NO:58, SEQ ID NO:61, SEQ ID NO:62, SEQ ID NO:63, SEQ ID NO:64, SEQ ID NO:66, SEQ ID NO:68, SEQ ID NO:68, SEQ ID NO:70 and SEQ ID NO:74, yet more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52 and SEQ ID NO:56.

For the purpose of the specification, Table 1 provides a list of secretion signal sequences of secretory proteins and their corresponding SEQ ID numbers, originating from *Bacillus subtilis* and *Bacillus amyloliquefaciens.* Further secretion signal sequences from secretory proteins originating from *Bacillus subtilis* are provided as SEQ ID numbers in Table 2.

**Table 1: Secretory proteins and their corresponding secretion signal sequences originating from Bacillus subtilis and Bacillus amyloliquefaciens**

| Secretory protein | Secretion signal sequence | |
|---|---|---|
| | SEQ ID NO *(Bacillus subtilis)* | SEQ ID NO *(Bacillus amyloliquefaciens)* |
| PhoA | 39 | 58 |
| YjfA | 40 | 59 |
| YckD | 41 | 60 |
| YbdN | 42 | 61 |
| YdjM | 43 | 62 |
| AbnA | 44 | 63 |
| YqgA | 45 | 64 |
| YbfO | 46 | - |
| YkvV/StoA | 47 | 65 |
| DacF | 48 | 66 |
| LytC | 49 | 67 |
| YncM | 50 | 68 |
| Vpr | 51 | 69 |
| YfkD | 52 | 70 |
| YpuA | 53 | 71 |
| YuaB | 54 | 72 |
| YvnB | 55 | 73 |
| YwtD/PgdS | 56 | 74 |
| YxaK/YxaL | 57 | 75 |

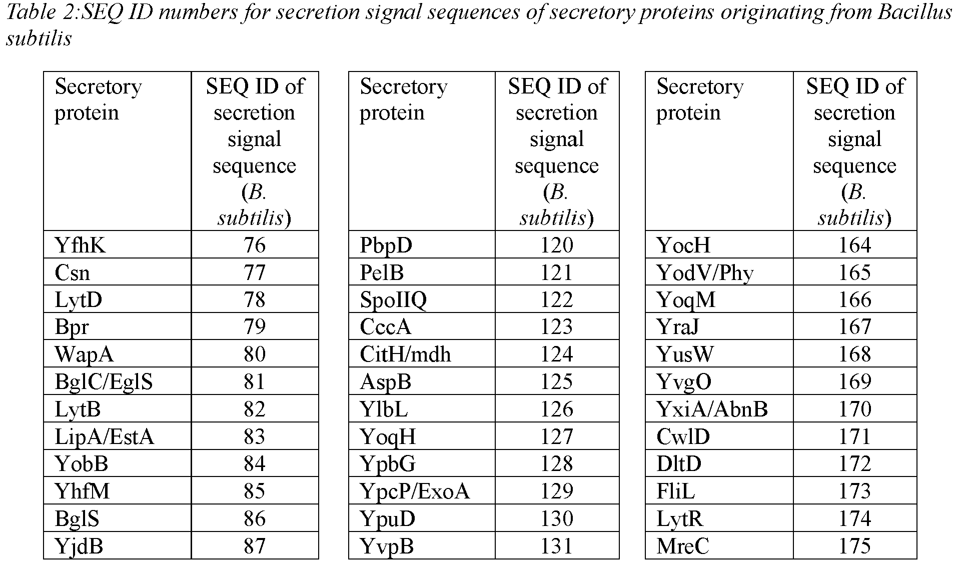

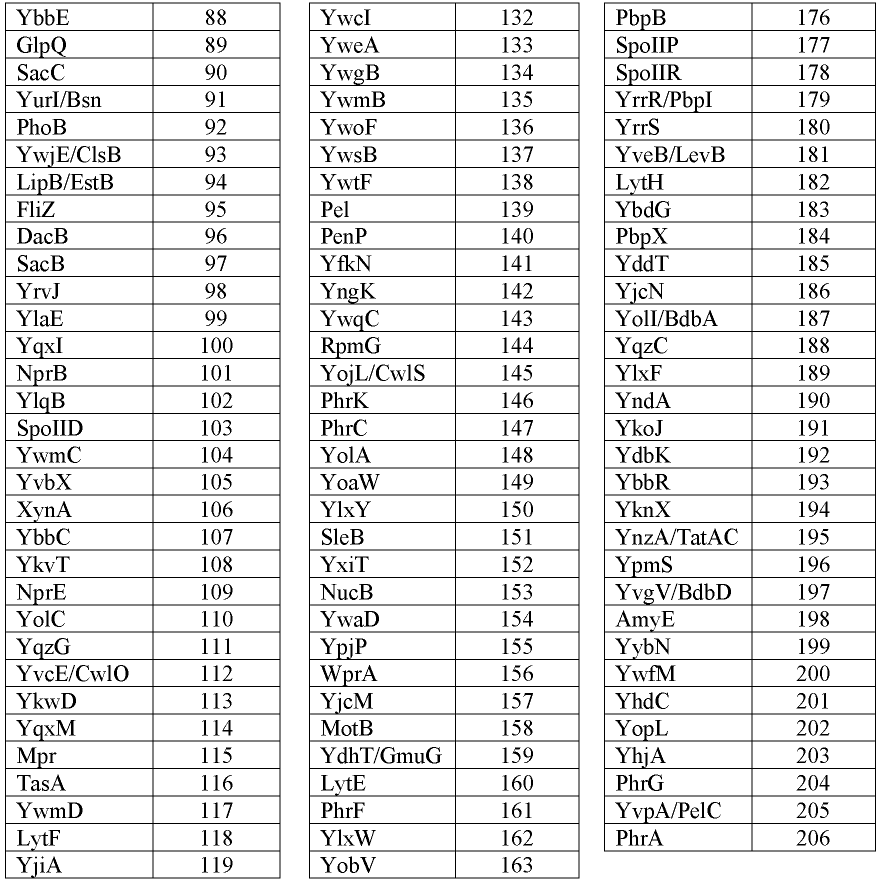

For the purpose of the specification, Table 3 provides a list of amino acid sequences and their corresponding SEQ ID numbers.

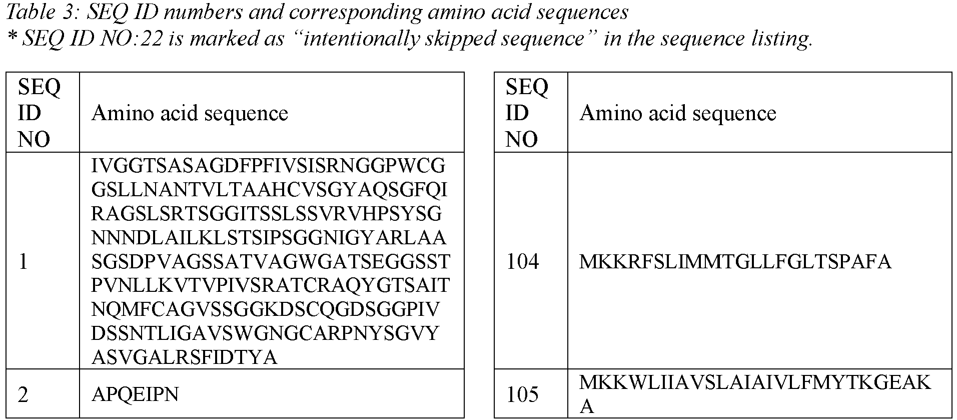

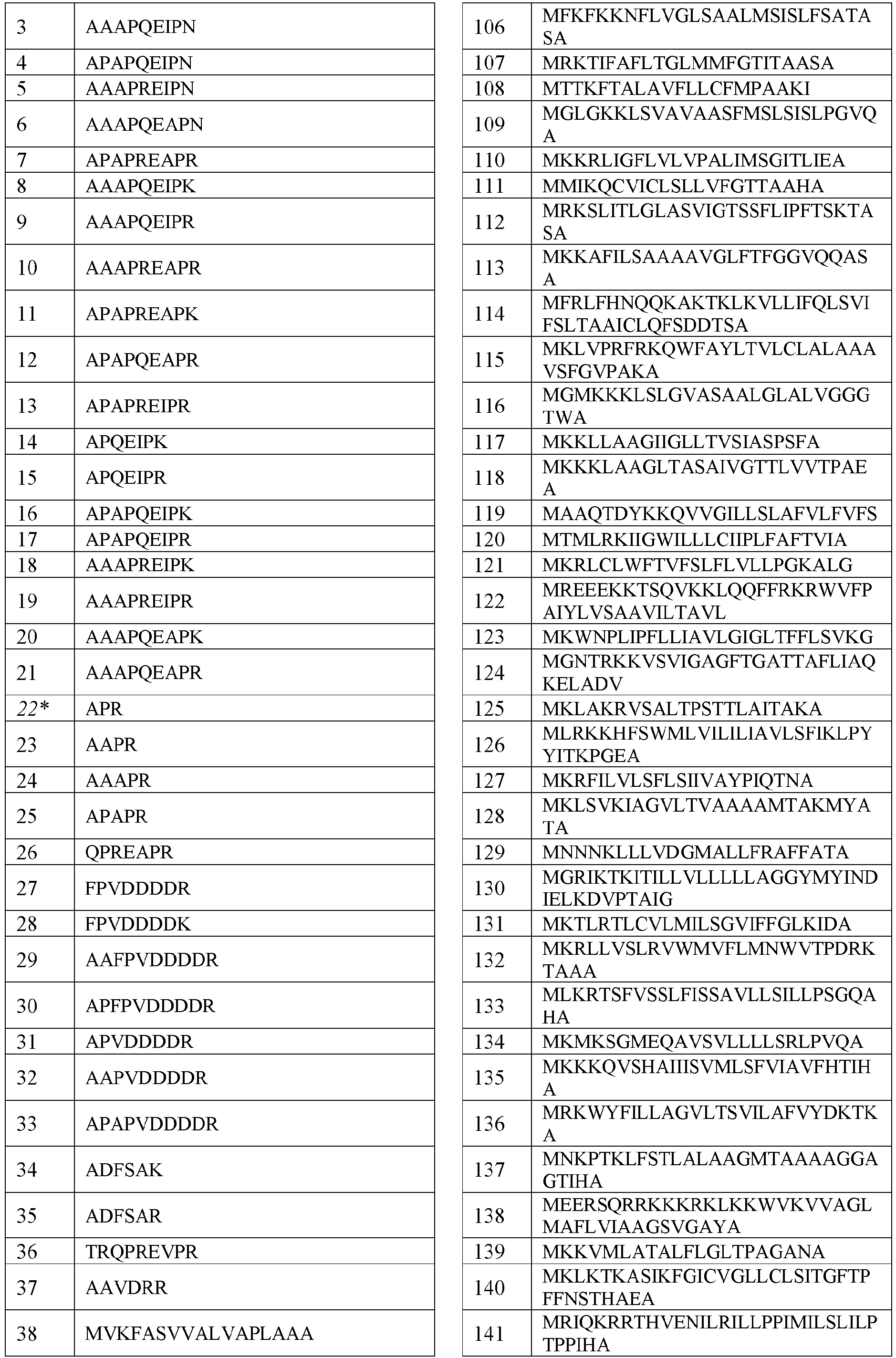

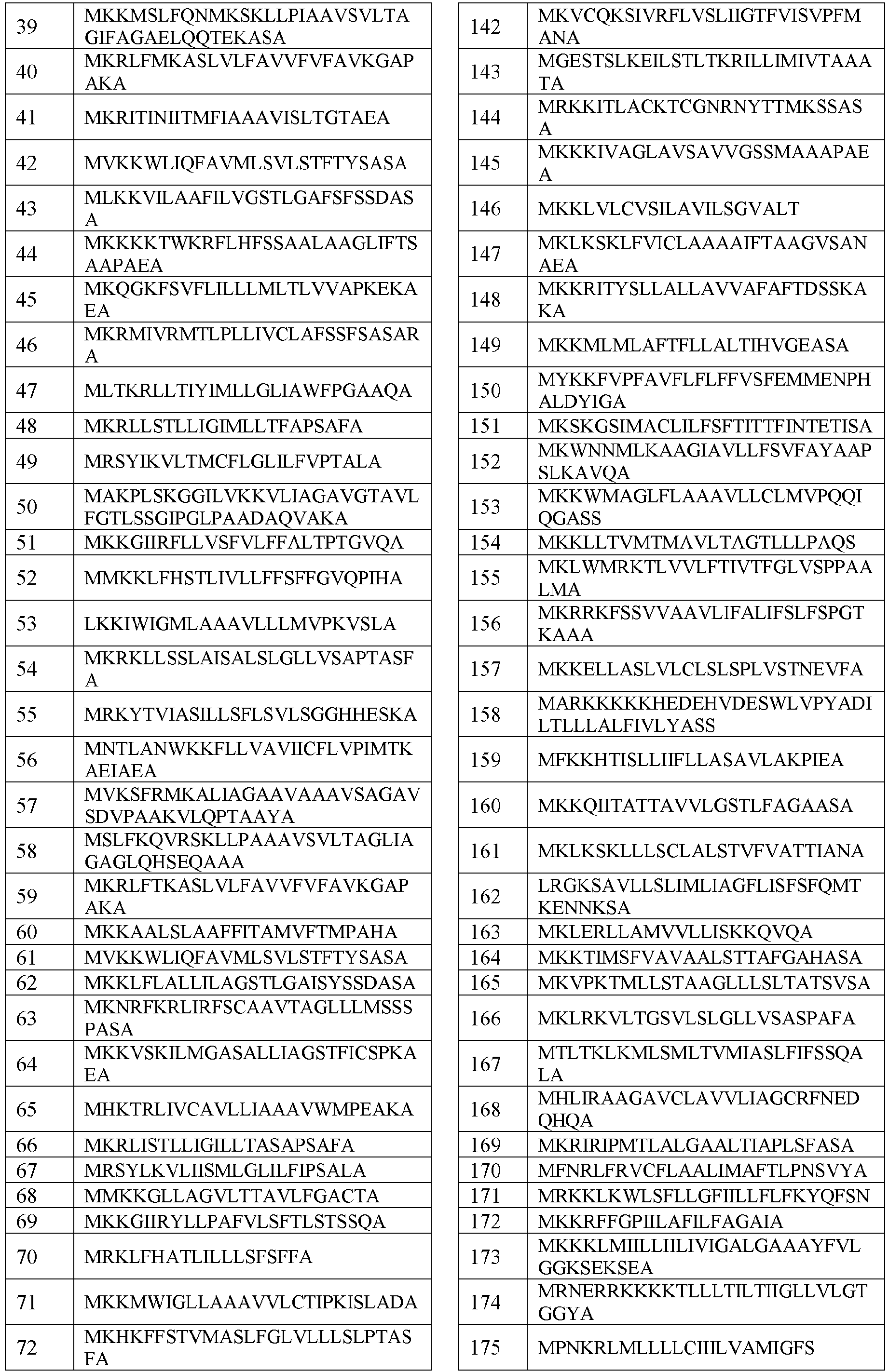

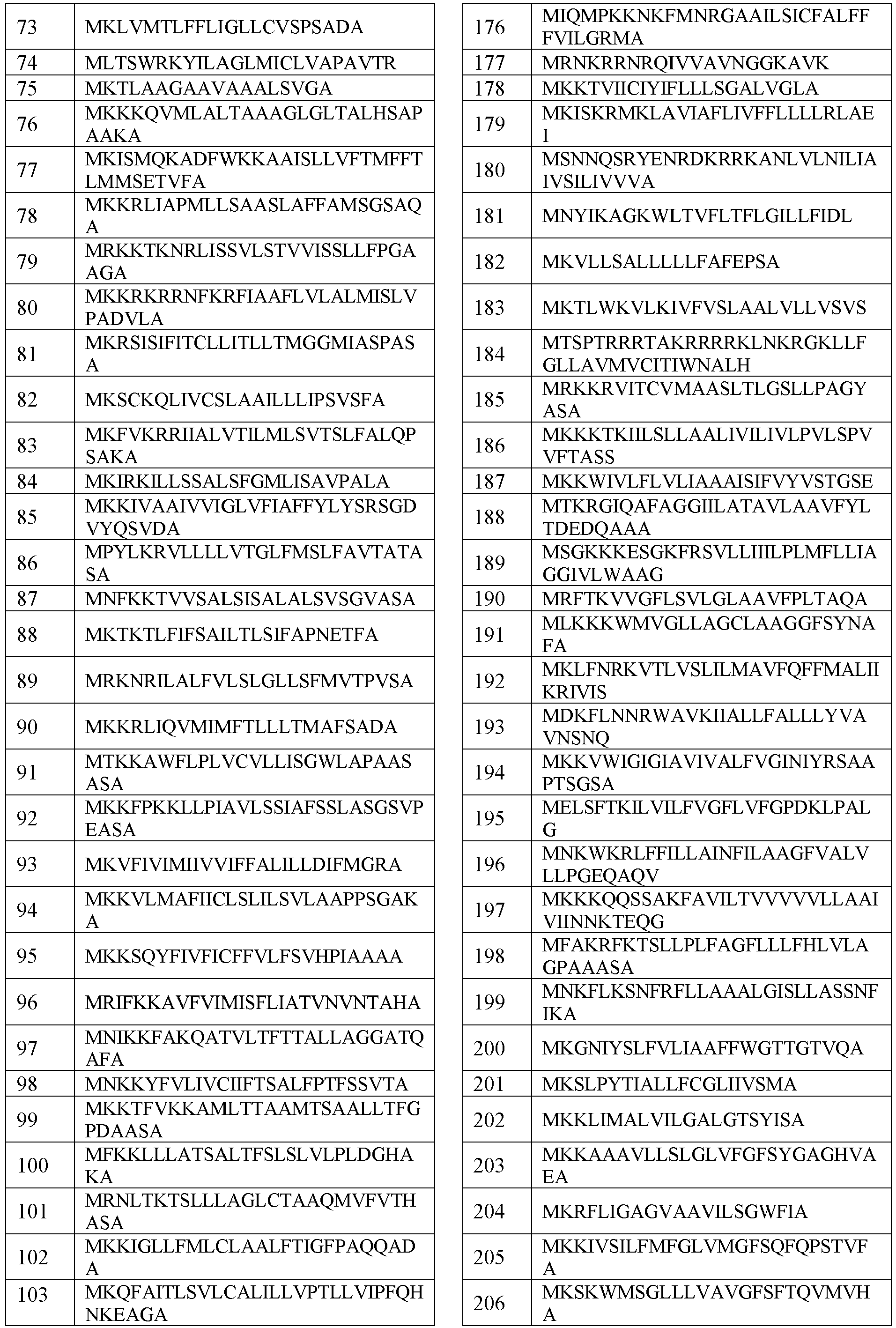

For the purpose of the specification, SEQ ID NO:22 could not be included in the sequence listing and is marked therein as "intentionally skipped sequence" due to requirement for a minimum sequence length of 4 amino acids. Any reference to SEQ ID NO:22 in the specification should refer to the amino acid sequence as listed in Table 3 (i.e., APR).

For the purpose of clarification, amino acid sequences of a certain protein from different species originating from the genus *Bacillus* contain conserved regions when aligned and this applies also to amino acid sequences of secretion signal sequences from a certain protein within the genus *Bacillus* like the species individualized above. Without wishing to be bound on any scientific theory, due to the conserved nature of these sequences and the specific motif-like structure of secretion signals, secretion signal sequences from one *Bacillus* species can be effectively used to traffic a protein in another *Bacillus* species. The conserved regions in the secretion signal sequences enable them to function across different species, facilitating the secretion and proper localization of proteins, even when originating from different *Bacillus* species. As a result, the sequence identity of these secretion signal sequences may vary, while still retaining their functional capacity across different *Bacillus* species due to the presence of conserved functional regions.

In preferred embodiments of the method according to the invention, step (a) involves processing the preproenzyme into a proenzyme upon expression and secretion, such that:
(i) the bacterial secretion signal sequence is cleaved off the preproenzyme during or after secretion from the *Bacillus* host cell; and
(ii) the resulting proenzyme is located in the culture medium and comprises the prosequence and the trypsin-like protease but not the bacterial secretion signal sequence.

In preferred embodiments of the method according to the invention, step (a) involves autocatalytically activating the proenzyme such that the prosequence is cleaved off from the trypsin-like protease thereby releasing the mature trypsin-like protease having trypsin-like activity.

Preferably, the autocatalytic activation takes places in the culture medium without previously applying rebuffering steps.

In preferred embodiments of the method according to the invention, the mature trypsin-like protease has a volumetric activity of at least 0.2 kU/l, preferably at least 0.4 kU/l, more preferably at least 0.6 kU/l, still more preferably at least 0.8 kU/l, still more preferably at least 1 kU/l, still more preferably at least 1.5 kU/l, still more preferably at least 2 kU/l, still more preferably at least 3 kU/l, still more preferably at least 4 kU/l, still more preferably at least 5 kU/l, still more preferably at least 6 kU/l, yet more preferably at least 7 kU/l, even more preferably at least 8 kU/l, most preferably at least 9 kU/l, in particular at least 10 kU/l.

In preferred embodiments of the method according to the invention, the mature trypsin-like protease has a recoverable volumetric activity of at least 0.2 kU/l, preferably at least 0.4 kU/l, more preferably at least 0.6 kU/l, still more preferably at least 0.8 kU/l, still more preferably at least 1 kU/l, still more preferably at least 1.5 kU/l, still more preferably at least 2 kU/l, still more preferably at least 3 kU/l, still more preferably y at least 4 kU/l, still more preferably at least 5 kU/l, still more preferably at least 6 kU/l, yet more preferably at least 7 kU/l, even more preferably at least 8 kU/l, most preferably at least 9 kU/l, in particular at least 10 kU/l.

In preferred embodiments of the method according to the invention, the mature trypsin-like protease exhibits
- an increased volumetric activity; and/or
- an increased volumetric activity recoverable;
compared to the mature trypsin-like protease prepared by the method according to the invention wherein the prosequence consists of SEQ ID NO:2 (wild-type).

Preferably, the volumetric activity is increased at least 1.1-fold, preferably at least 1.5-fold, more preferably at least 2-fold; still more preferably at least 5-fold, yet more preferably at least 10-fold, and even more preferably at least 20-fold.

Preferably, the volumetric activity recoverable is increased at least 1.1-fold, preferably at least 1.5-fold, more preferably at least 2-fold; still more preferably at least 5-fold, yet more preferably at least 10-fold, and even more preferably at least 20-fold.

Preferably, the volumetric activity or the volumetric activity recoverable of the trypsin-like protease is measured in the cell culture supernatant after 15 h to 65 h of expression from a cell culture supernatant having a pH preferably from about 6.0 to about 8.5, more preferably from about 6.3 to about 7.5 according to standard test conditions.

For the purpose of the specification, *"volumetric activity"* refers to the amount of enzymatic activity, the ability of the mature trypsin-like protease to catalyze a specific reaction, present in a given volume of the cell culture supernatant. The term *"volumetric activity recoverable"* is used to describe the volumetric activity calculated with biomass consideration. Biomass is determined as wet weight of cells (WCW [g/l]). The volumetric activity recoverable is calculated as follows: volumetric activity recoverable (kU/1) = volumetric activity (kU/l)/1000*(1000-WCW(g/l)). Volumetric activity and volumetric activity recoverable are typically expressed as units of activity per volume, such as kilo-units per liter (kU/l). Both values show the enzyme's functional efficiency in the culture medium and therefore can express the production yield of the enzyme in relation to the volume of the medium (with or without consideration of the biomass), reflecting the overall efficiency of the expression method.

For the purposes of the specification, *"standard test conditions"* refer to well-established specific experimental parameters and protocols under which the activity of the trypsin-like protease is measured, ensuring consistency and reproducibility. Preferably, the method involves the cleavage of the substrate N-alpha-acetyl-L-arginine-4-nitroanilide, resulting in the production of p-nitroaniline (p-NA), which leads to a color change in the reaction mixture to a yellow product. The absorbance of the product is preferably measured at 405 nm (A405). The standard test conditions preferably include using 8 mM of N-alpha-acetyl-L-arginine-4-nitroanilide in 100 mM Tris-HCl buffer at pH 8.0, with 1.5 mM CaCl2 and 0.225 g/l Brij, at a temperature of 37 °C. One unit of trypsin-like protease activity is defined as the amount of enzyme that cleaves the substrate to yield 1 µmol of p-nitroaniline per minute under these specified conditions. These standard test conditions ensure the accurate, consistent, and reproducible measurement of trypsin-like protease activity across different experiments. The techniques and conditions described herein are provided as illustrative examples. However, a skilled person in the field will appreciate how to modify and adapt these techniques and conditions based on specific requirements or varying circumstances. For the purpose of clarification, also other substances are suitable to measure trypsin activity, e.g., TAME (N-alpha-p-toluene-sulfonyl-L-arginine methyl ester) or BAEE (N-alpha-benzoyl-L-arginine ethyl ester).

In preferred embodiments of the method according to the invention, the host cell is selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis;* more preferably *B. amyloliquefaciens.*

In preferred embodiments of the method according to the invention, step (a) involves growing the host cell at a temperature range of about 28°C to about 37°C, preferably at about 30°C and in a medium with a pH range of about 6.0 to about 8.5, preferably with a pH range of about 6.3 to about 7.5.

In preferred embodiments of the method according to the invention, optional step (b) involves one or more of the following substeps:
- adjusting the pH of the supernatant;
- flocculation, preferably using a polycationic flocculant, preferably selected from the group consisting of polyethylenimines and polydiallyldimethyl ammonium chloride (pDADMAC), more preferably selected from Lupasol branched polyethyleneimines (PEI), Superfloc^{®} 781 G, Superfloc^{®} C448, Superfloc^{®} C581 G, Superfloc^{®} C752, Superfloc^{®} SD-2081;
- solid/liquid separation of cells from the culture medium using techniques such as, but not limited to, centrifugation, filtration, or sedimentation, thereby obtaining a cell culture supernatant; and/or
- concentrating the cell culture supernatant;
or any combination thereof.

In preferred embodiments of the method according to the invention, optional step (c) involves one or more of the following substeps:
- filtration; and/or
- chromatography such as anion exchange chromatography, hydrophobic interaction chromatography, size exclusion chromatography and/or affinity chromatography.

In preferred embodiments of the method according to the invention, optional step (d) comprises
- mixing the trypsin-like protease obtained in step (b) or step (c) with one or more stabilizers, preservatives, excipients or buffers, such as PBS and/or EDTA,
- adjusting the concentration of the trypsin-like protease to a desired level for its intended application; and/or;
- preparing the trypsin-like protease in a solid, liquid, or lyophilized form for storage, transport, or use.

In preferred embodiments of the method according to the invention, optional step (e) comprises placing the trypsin-like protease obtained in step (c) or step (d) in a suitable container for storage or distribution, preferably under sterile conditions.

A second aspect of the invention relates to a preproenzyme, preferably as defined according to any embodiment of the first aspect of the invention, comprising or consisting of
- a trypsin-like protease having a sequence identity of at least 70% compared to SEQ ID NO: 1;
- preferably, a prosequence located upstream of the trypsin-like protease and having at its C-terminus a basic amino acid residue, more preferably K or R; even more preferably wherein the prosequence consists of an amino acid sequence having a sequence identity of at least 70% compared to any of SEQ ID NO:7 to SEQ ID NO:37, yet more preferably compared to any of SEQ ID NO:7 to SEQ ID NO:26; and
- a bacterial secretion signal sequence located upstream of the trypsin-like protease and of the optionally present prosequence, wherein the bacterial secretion signal sequence is capable of directing secretion in a *Bacillus* host cell to a culture medium, preferably wherein the bacterial secretion signal sequence originates from *Bacillus.*

A third aspect of the invention relates to an expression vector comprising a DNA sequence coding for the preproenzyme according to the second aspect of the invention.

For the purpose of the specification, the term *"expression vector"* refers to a plasmid, virus, or other DNA construct that is designed to deliver a specific gene or DNA sequence into a host cell to enable the expression of the gene it carries. In this case, the expression vector contains a DNA sequence encoding the preproenzyme, allowing for its transcription and translation within the host cell. The expression vector typically includes regulatory elements necessary for the expression of the gene, such as a promoter, ribosome binding site, and terminator, along with any additional sequences required for selection, replication, or stability within the host organism. The expression vector serves as a tool to facilitate the efficient production of the desired preproenzyme within the host cell by enabling the gene to be transcribed and translated into its corresponding protein product.

A fourth aspect of the invention relates to a host cell comprising a DNA sequence coding for the preproenzyme according to the second aspect of the invention or the third aspect of the invention.

In a preferred embodiment of the host cell according to the invention, the DNA sequence coding for the preproenzyme is located on an expression vector.

In another preferred embodiment of the host cell according to the invention, the DNA sequence coding for the preproenzyme is integrated in the genome of the host cell.

In preferred embodiments of the host cell according to the invention, the host cell is a bacterium; preferably a gram-positive bacterium; more preferably a species selected from the genus *Bacillus;* still more preferably selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* yet more preferably *B. amyloliquefaciens, B. brevis, B. cereus, B. licheniformis, B. megaterium, B. pumilus,* and *B. subtilis;* even more preferably *B. amyloliquefaciens.*

Particularly preferred embodiments of the invention are compiled as clauses 1 to 70 hereinafter:
Clause 1. A method for producing a trypsin-like protease in Bacillus, the method comprising the steps of
   (a) expressing in a Bacillus host cell a gene coding for a preproenzyme comprising or consisting of
      - a trypsin-like protease having a sequence identity of at least 70% compared to SEQ ID NO:1;
      - preferably, a prosequence located upstream of the trypsin-like protease and having at its C-terminus a basic amino acid residue, more preferably K or R; and
      - a bacterial secretion signal sequence located upstream of the trypsin-like protease and of the optionally present prosequence, wherein the bacterial secretion signal sequence is capable of directing secretion in a Bacillus host cell to a culture medium, preferably wherein the bacterial secretion signal sequence originates from *Bacillus;*
      thereby providing a culture medium comprising the mature trypsin-like protease;
   (b) optionally, working-up the cell culture obtained in step (a) thereby providing a solid cell culture residue and a liquid cell culture supernatant;
   (c) optionally, purifying the mature trypsin-like protease from the cell culture supernatant;
   (d) optionally, formulating the mature trypsin-like protease; and
   (e) optionally, packaging the purified mature trypsin-like protease.
Clause 2. The method according to clause 1, wherein the prosequence consists of an amino acid sequence having a sequence identity of at least 70% compared to any of SEQ ID NO:7 to SEQ ID NO:37, preferably compared to any of SEQ ID NO:7 to SEQ ID NO:26.
Clause 3. The method according to clause 1 or 2, wherein the prosequence has at its N-terminus a aliphatic amino acid residue, preferably A.
Clause 4. The method according to any of clauses 1 to 3, wherein the prosequence consists of 10 amino acid residues.
Clause 5. The method according to any of clauses 1 to 3, wherein the prosequence consists of 9 amino acid residues.
Clause 6. The method according to any of clauses 1 to 3, wherein the prosequence consists of 8 amino acid residues.
Clause 7. The method according to any of clauses 1 to 3, wherein the prosequence consists of 7 amino acid residues.
Clause 8. The method according to any of clauses 1 to 3, wherein the prosequence consists of 6 amino acid residues.
Clause 9. The method according to any of clauses 1 to 3, wherein the prosequence consists of 5 amino acid residues.
Clause 10. The method according to any of clauses 1 to 3, wherein the prosequence consists of 4 amino acid residues.
Clause 11. The method according to any of clauses 1 to 3, wherein the prosequence consists of 3 amino acid residues.
Clause 12. The method according to any of the preceding clauses, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:7.
Clause 13. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:8.
Clause 14. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:9.
Clause 15. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 10.
Clause 16. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 11.
Clause 17. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:12.
Clause 18. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:13.
Clause 19. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 14.
Clause 20. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:15.
Clause 21. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:16.
Clause 22. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:17.
Clause 23. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO: 18.
Clause 24. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:19.
Clause 25. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:20.
Clause 26. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:21.
Clause 27. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to the amino acid sequence APR (also referred to as *"SEQ ID NO: 22*").
Clause 28. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:23.
Clause 29. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:24.
Clause 30. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:25.
Clause 31. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:26.
Clause 32. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:27.
Clause 33. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:28.
Clause 34. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:29.
Clause 35. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:30.
Clause 36. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:31.
Clause 37. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:32.
Clause 38. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:33.
Clause 39. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:34.
Clause 40. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:35.
Clause 41. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:36.
Clause 42. The method according to any of clauses 1 to 11, wherein the prosequence consists of an amino acid sequence having a sequence identity of at least 75%, preferably at least 80%, more preferably at least 85%, still more preferably at least 90%, yet more preferably at least 95%, even more preferably 100% compared to SEQ ID NO:37.
Clause 43. The method according to any of the preceding clauses, wherein the bacterial secretion signal is directly linked to the prosequence.
Clause 44. The method according to any of the preceding clauses, wherein the prosequence is directly linked to the trypsin-like protease.
Clause 45. The method according to any of the preceding clauses, wherein the bacterial secretion signal sequence originates from a species within the genus Bacillus selected from the group consisting *of B. amyloliquefaciens, B. paralicheniformis, B. aerius, B. aerophilus, B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus,* B. *globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis.*
Clause 46. The method according to any of the preceding clauses, wherein the bacterial secretion signal sequence is the secretion signal sequence from secretory *Bacillus* proteins selected from the group consisting of YjfA, YfhK, Csn, LytD, Bpr, WapA, BglC/EglS, LytB, LipA/EstA, YckD, YbdN, YobB, YhfM, BglS, YjdB, YbbE, GlpQ, SacC, YurI/Bsn, PhoB, YdjM, AbnA, YwjE/ClsB, YqgA, LipB/EstB, FliZ, DacB, SacB, YrvJ, YlaE, YqxI, NprB, YbfO, YlqB, SpoIID, YwmC, YvbX, YkvV/StoA, XynA, YbbC, YkvT, NprE, YolC, YqzG, YvcE/CwlO, YkwD, YqxM, Mpr, TasA, YwmD, DacF, LytF, YjiA, PbpD, PelB, SpoIIQ, CccA, CitH/mdh, AspB, YlbL, YoqH, YpbG, YpcP/ExoA, YpuD, YvpB, YwcI, YweA, YwgB, YwmB, YwoF, YwsB, YwtF, Pel, PenP, LytC, YfkN, YngK, YwqC, RpmG, YojL/CwlS, YncM, PhrK, Vpr, PhrC, YfkD, YolA, YoaW, YlxY, SleB, YxiT, NucB, YpuA, YwaD, YpjP, WprA, YjcM, MotB, YdhT/GmuG, LytE, PhrF, YlxW, YobV, YocH, YodV/Phy, YoqM, YraJ, YuaB, YusW, YvgO, YvnB, YxiA/AbnB, CwlD, DltD, FliL, LytR, MreC, PbpB, PhoA, SpoIIP, SpoIIR, YrrR/PbpI, YrrS, YveB/LevB, LytH, YbdG, PbpX, YddT, YjcN, YolI/BdbA, YqzC, YlxF, YndA, YkoJ, YwtD/PgdS, YdbK, YbbR, YknX, YnzA/TatAC, YpmS, YvgVBdbD, AmyE, YybN, YwfM, YhdC, YxaK/YxaL, YopL, YhjA, PhrG, YvpA/PelC and PhrA; preferably PhoA, YncM, YqgA, YjfA, YfkD, StoA, YdjM, DacF, Vpr, YbfO, AbnA, LytC, PgdS, YckD, YpuA, YuaB, YvnB, YxaL, YoqH and YbdN.
Clause 47. The method according to any of the preceding clauses, wherein step (a) involves bioinformatically determining the bacterial secretion signal sequence, preferably using bioinformatic tools selected from the group consisting of SignalP, Phobius, PrediSi, SIGCLEAVE, ANTHEPROT - Signal prediction, SOSUIsignal, Signal Find Server, SPD - Secreted Protein Database and SPEPlip. Clause 48. The method according to any of the preceding clauses, wherein the bacterial secretion signal sequence consists of an amino acid sequence having a sequence identity of at least 70% compared to any of SEQ ID NO:39 to SEQ ID NO:206, preferably to any of SEQ ID NO:39 to SEQ ID NO:75, more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52, SEQ ID NO:56, SEQ ID NO:58, SEQ ID NO:61, SEQ ID NO:62, SEQ ID NO:63, SEQ ID NO:64, SEQ ID NO:66, SEQ ID NO:68, SEQ ID NO:68, SEQ ID NO:70 and SEQ ID NO:74, yet more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52 and SEQ ID NO:56. Clause 49. The method according to clause 48, wherein sequence identity is at least 73%, preferably at least 75%, more preferably at least 77%, still more preferably at least 80%, yet more preferably at least 83%, even more preferably at least 85%, most preferably at least 87%, and in particular at least 90%, or at least 91%, preferably at least 92%, more preferably at least 93%, still more preferably at least 94%, yet more preferably at least 95%, even more preferably at least 96%, even more preferably at least 97%, even more preferably at least 98%, most preferably at least 99%, in particular100%.
Clause 50. The method according to any of the preceding clauses, wherein step (a) involves processing the preproenzyme into a proenzyme upon expression and secretion, such that:
   (i) the bacterial secretion signal sequence is cleaved off the preproenzyme during or after secretion from the *Bacillus* host cell; and
   (ii) the resulting proenzyme is located in the culture medium and comprises the prosequence and the trypsin-like protease.
Clause 51. The method according to any of the preceding clauses, wherein step (a) involves autocatalytically activating the proenzyme such that the prosequence is cleaved off from the trypsin-like protease thereby releasing the mature trypsin-like protease having trypsin-like activity.
Clause 52. The method according to any of the preceding clauses, wherein the autocatalytic activation takes places in the culture medium without rebuffering steps.
Clause 53. The method according to any of the preceding clauses, wherein the mature trypsin-like protease has a volumetric activity of at least 0.2 kU/l, preferably at least 0.4 kU/l, more preferably at least 0.6 kU/l, still more preferably at least 0.8 kU/l, still more referably at least 1 kU/l, still more preferably at least 1.5 kU/l, still more preferably at least 2 kU/l, still more preferably at least 3 kU/l, still more preferably at least 4 kU/l, still more preferably at least 5 kU/l, still more preferably at least 6 kU/l, yet more preferably at least 7 kU/l, even more preferably at least 8 kU/l, most preferably at least 9 kU/l, in particular at least 10 kU/l.
Clause 54. The method according to any of the preceding clauses, wherein the mature trypsin-like protease has a recoverable volumetric activity of at least 0.2 kU/l, preferably at least 0.4 kU/l, more preferably at least 0.6 kU/l, still more preferably at least 0.8 kU/l, still more preferably at least 1 kU/l, still more preferably at least 1.5 kU/l, still more preferably at least 2 kU/l, still more preferably at least 3 kU/l, still more preferably at least 4 kU/l, still more preferably at least 5 kU/l, still more preferably at least 6 kU/l, yet more preferably at least 7 kU/l, even more preferably at least 8 kU/l, most preferably at least 9 kU/l, in particular at least 10 kU/l.
Clause 55. The method according to any of the preceding clauses, wherein the mature trypsin-like protease exhibits
   - an increased volumetric activity; and/or
   - an increased volumetric activity recoverable;
   compared to the mature trypsin-like protease prepared by the method according to clause 1 wherein the prosequence consists of SEQ ID NO:2 (wild-type).
Clause 56. The method according to any of the preceding clauses, wherein the volumetric activity is increased at least 1.1-fold, preferably at least 1.5-fold, more preferably at least 2-fold; still more preferably at least 5-fold, yet more preferably at least 10-fold, and even more preferably at least 20-fold.
Clause 57. The method according to any of the preceding clauses, wherein the volumetric activity recoverable is increased at least 1.1-fold, preferably at least 1.5-fold, more preferably at least 2-fold; still more preferably at least 5-fold, yet more preferably at least 10-fold, and even more preferably at least 20-fold.
Clause 58. The method according to any of the preceding clauses, wherein the volumetric activity or the volumetric activity recoverable of the trypsin-like protease is measured in the cell culture supernatant having a pH preferably from about 6.0 to about 8.5, more preferably from about 6.3 to about 7.5 according to standard test conditions.
Clause 59. The method according to any of the preceding clauses, wherein the host cell is selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis;* more preferably *B. amyloliquefaciens.*
Clause 60. The method according to any of the preceding clauses, wherein step (a) involves growing the host cell at a temperature range of about 28°C to about 37°C, preferably at about 30°C and in a medium with a pH range of about 6.0 to about 8.5, preferably with a pH range of about 6.3 to about 7.5.
Clause 61. The method according to any of the preceding clauses, wherein optional step (b) involves one or more of the following substeps:
   - adjusting the pH of the supernatant;
   - flocculation, preferably using a polycationic flocculant, preferably selected from the group consisting of polyethylenimines and polydiallyldimethyl ammonium chloride (pDADMAC), more preferably selected from Lupasol branched polyethyleneimines (PEI), Superfloc^{®} 781 G, Superfloc^{®} C448, Superfloc^{®} C581 G, Superfloc^{®} C752, Superfloc^{®} SD-2081;
   - solid/liquid separation of cells from the culture medium using techniques such as, but not limited to, centrifugation, filtration, or sedimentation, thereby obtaining a cell culture supernatant; and/or
   - concentrating the cell culture supernatant;
   or any combination thereof.
Clause 62. The method according to any of the preceding clauses, wherein optional step (c) involves one or more of the following substeps:
   - filtration; and/or
   - chromatography such as anion exchange chromatography, hydrophobic interaction chromatography, size exclusion chromatography and/or affinity chromatography.
Clause 63. The method according to any of the preceding clauses, wherein optional step (d) comprises
   - mixing the mature trypsin-like protease obtained in step (b) or step (c) with one or more stabilizers, preservatives, excipients or buffers such as PBS and/or EDTA,
   - adjusting the concentration of the trypsin-like protease to a desired level for its intended application; and/or;
   - preparing the mature trypsin-like protease in a solid, liquid, or lyophilized form for storage, transport, or use.
Clause 64. The method according to any of the preceding clauses, wherein optional step (e) comprises placing the mature trypsin-like protease obtained in step (c) or step (d) in a suitable container for storage or distribution, preferably under sterile conditions.
Clause 65. A preproenzyme, preferably as defined in any of clauses 1 to 58, comprising or consisting of
   - a trypsin-like protease having a sequence identity of at least 70% compared to SEQ ID NO: 1;
   - preferably, a prosequence located upstream of the trypsin-like protease and having at its C-terminus a basic amino acid residue, more preferably K or R; even more preferably wherein the prosequence consists of an amino acid sequence having a sequence identity of at least 70% compared to any of SEQ ID NO:7 to SEQ ID NO:37, yet more preferably compared to any of SEQ ID NO:7 to SEQ ID NO:26; and
   - a bacterial secretion signal sequence located upstream of the prosequence, wherein the bacterial secretion signal sequence is capable directing secretion in a *Bacillus* host cell, preferably which originates from *Bacillus.*
Clause 66. An expression vector comprising a DNA sequence coding for the according to clause 65.
Clause 67. A host cell comprising a DNA sequence coding for the preproenzyme according to clause 65.
Clause 68. The host cell according to clause 67, wherein the DNA sequence coding for the preproenzyme is located on an expression vector.
Clause 69. The host cell according to clause 67, wherein the DNA sequence coding for the preproenzyme is integrated in the genome of the host cell.
Clause 70. The host cell according to any of clauses 67 to 69, wherein the host cell is a bacterium; preferably a gram-positive bacterium; more preferably a species selected from the genus *Bacillus;* still more preferably selected from the group consisting of *B. amyloliquefaciens, B. paralicheniformis, B. aerius, B. aerophilus, B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and B. *vireti;* yet more preferably *B. amyloliquefaciens, B. brevis, B. cereus, B. licheniformis, B. megaterium, B. pumilus,* and *B. subtilis;* even more preferably *B. amyloliquefaciens.*

The following examples further illustrate the invention but are not to be construed as limiting its scope.

### Example 1: Design of different preproenzymes of trypsin-like protease and cloning for expression

All cloning and genetical engineering approaches used in this example were conducted using standard techniques known to the skilled person and described in the state of the art.

The gene coding for the wild-type preproenzyme of the trypsin-like protease of *Fusarium oxysporum,* was codon optimized for the expression in *Bacillus amyloliquefaciens* and synthesized by Eurofins MWG. The amino acid sequence of the wild-type preproenzyme of *Fusarium oxysporum* trypsin-like protease consists from the N-terminus to the C-terminus of the signal sequence of SEQ ID NO:38, the prosequence of SEQ ID NO:2 and the trypsin-like protease of SEQ ID NO:1. The corresponding DNA sequence was codon-optimized for the expression in *Bacillus* and cloned into the expression vector pLE2D06 (derivative of pSM19035, a low-copy-number theta-replicating plasmid well described in literature (Lioy et. al., Plasmid, 2010, doi: 10.1016/j.plasmid.2010.04.002)), having a growth phase induced neutral protease promoter (Pnpr) and a chloramphenicol resistance gene for selection.

Variants of the preproenzyme of the *Fusarium oxysporum* trypsin-like protease were constructed using codon-optimized DNA sequences for *Bacillus* expression, coding for different signal sequences and prosequences as illustrated in Table 4 and Table 5. The heterologous signal- and prosequences were cloned upstream of the DNA sequence coding for the trypsin-like protease of SEQ ID NO:1, each to replace the corresponding native signal- and prosequence of *Fusarium oxysporum* trypsin-like protease preproenzyme. All DNA sequences of the variants were cloned into the expression vector pLE2D06.

**Table 4: Variation of prosequences - Variants of the preproenzyme of the trypsin-like protease (SEQ ID NO:1) were constructed using the Bacillus subtilis signal sequence of the secretory protein alkaline phosphatase - PhoA (SEQ ID NO:39) located upstream of different prosequence variants and without a prosequence. As a control, the native secretion signal sequence of Fusarium oxysporum trypsin-like protease (SEQ ID NO:38) was tested with two different prosequence variants and without a prosequence.**

| Variant | Signal sequence | | | Prosequence |
|---|---|---|---|---|
| | Protein name | origin | SEQ ID NO: | SEQ ID NO: |
| 1 | Trypsin-like protease | *F. oxysporum* | 38 | 2 |
| 2 | Trypsin-like protease | *F. oxysporum* | 38 | - |
| 3 | Trypsin-like protease | *F. oxysporum* | 38 | 14 |
| 4 | PhoA | *B. subtilis* | 39 | 2 |
| 5 | PhoA | *B. subtilis* | 39 | 3 |
| 6 | PhoA | *B. subtilis* | 39 | 4 |
| 7 | PhoA | *B. subtilis* | 39 | 5 |
| 8 | PhoA | *B. subtilis* | 39 | 6 |
| 9 | PhoA | *B. subtilis* | 39 | - |
| 10 | PhoA | *B. subtilis* | 39 | 14 |
| 11 | PhoA | *B. subtilis* | 39 | 15 |
| 12 | PhoA | *B. subtilis* | 39 | 8 |
| 13 | PhoA | *B. subtilis* | 39 | 9 |
| 14 | PhoA | *B. subtilis* | 39 | 10 |
| 15 | PhoA | *B. subtilis* | 39 | 11 |
| 16 | PhoA | *B. subtilis* | 39 | 7 |
| 17 | PhoA | *B. subtilis* | 39 | 12 |
| 18 | PhoA | *B. subtilis* | 39 | 13 |

**Table 5: Variation of secretion signal sequences - Variants of the preproenzyme of the trypsin-like protease (SEQ ID NO:1) were constructed using different Bacillus secretion signal sequences from Bacillus subtilis located upstream of the prosequence of SEQ ID NO:7**

| Variant | Signal sequence | | Prosequence |
|---|---|---|---|
| | Name of secretory *Bacillus* protein | SEQ ID NO: | SEQ ID NO: |
| 16 | PhoA | 39 | 7 |
| 19 | YjfA | 40 | 7 |
| 20 | YckD | 41 | 7 |
| 21 | YbdN | 42 | 7 |
| 22 | YdjM | 43 | 7 |
| 23 | AbnA | 44 | 7 |
| 24 | YqgA | 45 | 7 |
| 25 | YbfO | 46 | 7 |
| 26 | YkvV/StoA | 47 | 7 |
| 27 | DacF | 48 | 7 |
| 28 | LytC | 49 | 7 |
| 29 | YncM | 50 | 7 |
| 30 | Vpr | 51 | 7 |
| 31 | YfkD | 52 | 7 |
| 32 | YpuA | 53 | 7 |
| 33 | YuaB | 54 | 7 |
| 34 | YvnB | 55 | 7 |
| 35 | YwtD/PgdS | 56 | 7 |
| 36 | YxaK/YxaL | 57 | 7 |

### Example 2: Expression of a trypsin-like protease preproenzyme in Bacillus

Variants of trypsin-like protease preproenzymes from *Fusarium oxysporum* as referenced in Example 1 (Table 4 and Table 5) were routinely expressed using *Bacillus amyloliquefaciens* (derivative of commercially available *Bacillus amyloliquefaciens* strain ATCC^{®} 23844^{™}) cells in 96-deep-well plates and/or shaking flasks by inoculation of the complex medium M1 (10 % maltose, 0.1 % glucose, 2 % nitrogen source, 2.5 % soluble corn polypeptides, 0.5 % ammonium source, 0.1 % kalium source, 0.05 % magnesium source) with fresh precultures. For the cultivation of precultures LB Medium (10 g/l tryptone, 5 g/l yeast extract, 10 g/l NaCl, pH 7.0) was supplemented with 1 % glucose and 10 µg/ml chloramphenicol. The precultures were grown at 30 °C until the cells reached the middle exponential phase. The expression cultures were inoculated at an initial optical density of 0.1 and grown at 30 °C. In 96-deep-well plates (DWP), 1 ml expression cultures were agitated at 900 rpm for a maximum of 46 hours. In 100 ml shake flasks, 20 ml cultures were agitated at 200 rpm for a maximum of 65 hours.

The trypsin-like protease activity was analyzed at time points ranging from 15 h to 65 h. Therefore, samples were withdrawn at indicated timepoints and culture supernatants containing the secreted trypsin-like protease were separated from cells by centrifugation. When the expression was analyzed in DWP, 200 µl of the expression culture were transferred in 96-well-microplates and centrifuged for 20 min at 3200 x g, 4 °C. When the expression was analyzed in shaking flasks, 1 ml expression culture were transferred in 1.5 ml tubes and centrifuged for 20 min at 16,200 x g and 4 °C. The obtained cell culture supernatants were separated from the pelleted cells, stored at -20 °C or used directly for determination of trypsin-like protease activity.

### Example 3: Measurement of trypsin-like protease activity

The trypsin-like protease activity was measured in cell culture supernatants obtained according to Example 2.

The assay for the detection of trypsin-like protease activity is based on the cleavage of the substrate N-alpha-acetyl-L-arginine-4-nitroanilide to generate p-nitroaniline (p-NA). The cleavage results in a color change of the reaction mixture to a yellow product. The absorbance of the product is then measured at 405 nm (A405). Increasing levels of p-nitroaniline cause a linear increase in absorbance, thus trypsin-like protease activity can be accurately measured. Quantification is achieved using a p-nitroaniline calibration curve. The linear range of the assay is between 0.02 and 0.2 mM p-nitroaniline, which corresponds to an approximate amount of 0.002 - 0.04 U of trypsin-like protease per 1 mL assay.

The activity measurements were performed in 96-well microplate format with a final volume of 200 µl. Substrate cleavage was initiated by adding 20 µl of various trypsin dilutions in assay buffer (100 mM Tris-HCl pH 8.0, 1.5 mM CaCl₂, 0.225 g/l Brij) to 180 µl reaction mixture prewarmed to 37 °C. As a control, 20 µl of assay buffer was added. The absorbance was read at 405 nm at 1-minute intervals for 15-30 minutes for each sample and blank.

One unit is defined as the amount of trypsin-like protease that cleaves the substrate yielding 1 µmol of p-nitroaniline per minute at 37 °C. The reaction mixture consists of 8 mM of N-alpha-acetyl-L-arginine-4-nitroanilide in 100 mM Tris-HCl pH 8.0, 1.5 mM CaCl₂ and 0.225 g/l Brij.

The activity of the trypsin-like protease was expressed as:
- Volumetric activity given in "kU/l" (kilo Units per liter); and
- Volumetric activity recoverable given in "kU/l" (kilo Units per liter).

The term "volumetric activity supernatant" refers to volumetric activity measured in culture supernatant without taking biomass into account.

The term "volumetric activity recoverable" is used to describe the volumetric activity calculated with biomass consideration. Biomass is determined as wet weight of cells (WCW [g/l]). Therefore, at specified intervals, 1 ml samples were extracted from the expression cultures as duplicates and centrifuged at 16.200 g for 20 min at 4 °C. The cell culture supernatant was removed, and the cell pellet was weighted to determine the wet weight. The volumetric activity recoverable is calculated as follows:
- volumetric activity recoverable (kU/l) = volumetric activity (kU/l)/1000*(1000-WCW(g/l)).

### Example 4: Variation of the prosequence

The preproenzyme variants according to Table 4 were expressed in shake flasks for 21 h up to 45 h according to Example 2. The activity of the trypsin-like protease was measured in the cell culture supernatant at indicated timepoints and volumetric activity or volumetric activity recoverable was measured according to Example 3 and is shown in Table 6 and Table 7.

**Table 6: Measurement of volumetric activity [kU/l] of the trypsin-like protease after 24 h and 47 h of expression in shake flasks using Variants 1 to 3 comprising a non-bacterial, native signal sequence from Fusarium oxysporum trypsin-like protease**

| Vari ant | Signal sequence | | | prosequ ence | WCW [g/l] | | volumetric activity [kU/l] | |
|---|---|---|---|---|---|---|---|---|
| | Name | origin | SEQ ID NO: | SEQ ID NO: | 24 h | 47 h | 24 h | 47 h |
| 1 | *F. oxysporum* Trypsin-like protease | *F. oxyspor um* | 38 | 2 | 70 | 96 | 0.0197 | 0.0481 |
| 2 | *F. oxysporum* Trypsin-like protease | *F. oxyspor um* | 38 | - | 82 | 119 | 0.0235 | 0.0572 |
| 3 | *F. oxysporum* Trypsin-like protease | *F. oxyspor um* | 38 | 14 | 79 | 116 | 0.0240 | 0.0458 |
| Control (empty plasmid) | | | | | 108 | 109 | 0.0176 | 0.0159 |

The variants 1 to 3 (according to Table 4) comprising the native, non-bacterial *Fusarium oxysporum* signal sequence did not show any trypsin-like activity significantly higher than for the control (empty plasmid) in the cell culture supernatant after 24 h and 47 h of expression in shake flasks. The WCW was monitored throughout the cultivation and showed a comparable cell growth for all variants.

**Table 7: Measurement of volumetric activity recoverable [kU/l] of the trypsin-like protease after 39 h and 45 h of expression in shake flasks and enhancement of volumetric activity recoverable compared to Variant 4 (native Fusarium oxysporum comprising the prosequence consisting of SEQ ID NO:2)**

| Variant | prosequence | 39 h | | 45 h | |
|---|---|---|---|---|---|
| | | vol. activity recoverable | | vol. activity recoverable | |
| | SEQ ID NO: | [kU/l] | Fold-enhancement to variant 4 | [kU/l] | Fold-enhancement to variant 4 |
| 4 | 2 | 2.2 | 1.0 | 2.7 | 1.0 |
| 5 | 3 | 2.3 | 1.0 | 2.9 | 1.1 |
| 6 | 4 | 1.0 | 0.4 | 1.5 | 0.66 |
| 7 | 5 | 2.2 | 1.0 | 3.5 | 1.3 |
| 8 | 6 | 1.6 | 0.7 | 2.3 | 0.8 |
| 9 | - | 25.0 | 11.2 | 26.0 | 9.5 |
| 10 | 14 | 34.1 | 15.2 | 38.7 | 14.2 |
| 11 | 15 | 41.5 | 18.6 | 44.0 | 16.2 |
| 12 | 8 | 48.3 | 21.65 | 45. 8 | 16.8 |
| 13 | 9 | 64.2 | 28.75 | 70.0 | 25.7 |
| 14 | 10 | 41.3 | 18.5 | 46.3 | 17.0 |
| 15 | 11 | 51.4 | 23.0 | 62.2 | 22.8 |
| 16 | 7 | 56.3 | 25.2 | 61.2 | 22.5 |
| 17 | 12 | 53.7 | 24.0 | 58.1 | 21.32 |
| 18 | 13 | 55.8 | 24.97 | 65.6 | 24.1 |
| Control (empty plasmid) | | 0.05 | 0.02 | 0.09 | 0.03 |

The data in Tables 6 and 7 indicate that expression of the trypsin-like protease as a preproenzyme comprising a bacterial secretion signal sequence located upstream of the trypsin-like protease has advantages compared to the native non-bacterial secretion signal sequence of *Fusarium oxysporum* in order to achieve an improved volumetric activity of the trypsin-like protease in the cell culture supernatant. This improvement is observed, even if the prosequence is not present. Activation of the trypsin-like protease preproenzyme has been shown for variants 4-8 comprising a prosequence with a C-terminal amino acid N but leading to relatively low volumetric activities recoverable from the cell culture supernatant. The data in Table 7 demonstrate that expression of the trypsin-like protease as a preproenzyme comprising artificial prosequences having at its C-terminus a basic amino acid residue of K or R (variants 10 - 18) improve the volumetric activity recoverable compared to the preproenzyme of variant 4 comprising the native prosequence of *Fusarium oxysporum* trypsin-like protease preproenzyme after 39 and 45 h of cultivation. The volumetric activity recoverable is even more enhanced after 39 and 45 h of cultivation when expressing the preproenzyme variants 12 to 18 (comprising prosequences of SEQ ID NO:7 to 13).

### Example 5: Variation of the secretion signal sequence

The variants according to Example 1, Table 5 were expressed in 96-deep-well plates as described in Example 2 to screen for further secretion signal sequences from *Bacillus* resulting in expression and activation of the trypsin-like protease preproenzyme. Therefore, the prosequence was not changed. The prosequence of SEQ ID NO:7 was constant for all variants. The activity of the trypsin-like protease was measured in the cell culture supernatant after 45 h of expression and volumetric activity was determined as described in Example 3. The volumetric activities of the trypsin-like protease measured from expression in DWP are not directly comparable to the activities obtained after expression in shake flasks. Hence, the variant 16 which was analyzed in Example 4 was used as a positive control.

**Table 8: Volumetric activities [kU/l] of trypsin-like protease preproenzyme variants 19 to 36 after 45 h of expression in DWP and comparison to variant 16 as positive control.**

| Variant | Signal sec uence | | vol. activity [kU/l] | vol. activity compared to variant 16 [%] |
|---|---|---|---|---|
| | Secretory protein | SEQ ID NO: | | |
| 16 | PhoA | 39 | 10.1 | = 100.0 |
| 19 | YjfA | 40 | 0.1 | 1.0 |
| 20 | YckD | 41 | 7.6 | 75.5 |
| 21 | YbdN | 42 | 8.1 | 80.4 |
| 22 | YdjM | 43 | 13.1 | 130.1 |
| 23 | AbnA | 44 | 8.6 | 84.8 |
| 24 | YqgA | 45 | 11.3 | 111.7 |
| 25 | YbfO | 46 | 8.0 | 79.7 |
| 26 | YkvV/StoA | 47 | 7.3 | 72.3 |
| 27 | DacF | 48 | 10.9 | 107.7 |
| 28 | LytC | 49 | 8.0 | 79.2 |
| 29 | YncM | 50 | 8.2 | 81.6 |
| 30 | Vpr | 51 | 10.4 | 102.8 |
| 31 | YfkD | 52 | 9. 7 | 95.9 |
| 32 | YpuA | 53 | 6.3 | 62.1 |
| 33 | YuaB | 54 | 7.1 | 70.3 |
| 34 | YvnB | 55 | 7.9 | 78.2 |
| 35 | YwtD/PgdS | 56 | 9.9 | 98.6 |
| 36 | YxaK/YxaL | 57 | 7.0 | 69.2 |
| Control - empty plasmid | | | 0.03 | 0.3 |

To verify the results obtained from expression in DWP, nine variants displayed in Table 8 were selected and expressed in shake flasks according to Example 2. The activity of the trypsin-like protease was measured in the cell culture supernatant after 22 h, 41 h, 46.5 h and 65 h of cultivation. Volumetric activity and volumetric activity recoverable were determined according to Example 3. The variant 16 was again used as a positive control for reference.

**Table 9: Volumetric activities and volumetric activities recoverable [kU/l] of indicated variants measured after 22 h, 41 h, 46.5 h and 65 h of cultivation.**

| Varia nt | Signal sequence | | 22 h [kU/l] | | 41 h [kU/l] | | 46.5 h [kU/l] | | 65 h [kU/l] | |
|---|---|---|---|---|---|---|---|---|---|---|
| | Secretory protein | SEQ ID NO: | vol. activi ty | vol. activity recovera ble | vol. activi ty | vol. activity recovera ble | vol. activi ty | vol. activity recovera ble | vol. activi ty | vol. activity recovera ble |
| 16 | PhoA | 39 | 16.1 | 14. 7 | 51.4 | 43.3 | 55.1 | 47.5 | 60.2 | 51.2 |
| 19 | YjfA | 40 | 11.7 | 10.2 | 39.6 | 33.6 | 41.9 | 35.8 | 45.9 | 38.7 |
| 22 | YdjM | 43 | 6.6 | 5.7 | 18.0 | 15.8 | 21.4 | 18.6 | 28. 7 | 23.5 |
| 24 | YqgA | 45 | 10.6 | 9.8 | 29.8 | 26.2 | 35.5 | 30.8 | 49.6 | 44.6 |
| 25 | YbfO | 46 | 4.5 | 4.3 | 10.0 | 9.1 | 10.3 | 9.1 | 12.3 | 10.9 |
| 26 | YkvV/St oA | 47 | 5.7 | 5.2 | 14.6 | 12.6 | 18.2 | 16.0 | 29.9 | 25.7 |
| 27 | DacF | 48 | 10.3 | 9.5 | 19.6 | 17.1 | 22.3 | 19. 9 | 25.5 | 22.1 |
| 29 | YncM | 50 | 13.0 | 11.6 | 32.6 | 27.2 | 41.4 | 35.4 | 54.9 | 47.1 |
| 30 | Vpr | 51 | 6.3 | 5.8 | 16.7 | 15.1 | 18.9 | 17.0 | 25.0 | 22.0 |
| 31 | YfkD | 52 | 10.1 | 9.2 | 24.4 | 20.5 | 29.4 | 25.2 | 38.9 | 33.3 |
| Control - empty plasmid | | | 0.16 | 0.11 | 0.20 | 0.11 | 0.20 | 0.11 | 0.23 | 0.14 |

The above data in Table 8 and Table 9 demonstrate that expression of the trypsin-like protease preproenzyme is not limited to the secretion signal sequence of SEQ ID NO:39. Comparable volumetric activities (recoverable) of the trypsin-like protease can be achieved using other secretion signal sequences capable of directing secretion in a *Bacillus* host cell to a culture medium and a prosequence which enables an efficient activation of the proenzyme to the mature trypsin-like protease. Efficient activation of the proenzyme to the mature trypsin-like protease was demonstrated in Example 4 for the prosequences of any of SEQ ID NO:7 to SEQ ID NO: 15, which are plausible to be combined with any of secretion signal sequences, preferably of any of SEQ ID NO:39 to SEQ ID NO:57.

## Claims

1. A method for producing a trypsin-like protease in *Bacillus,* the method comprising the steps of
(a) expressing in a *Bacillus* host cell a gene coding for a preproenzyme comprising or consisting of
- a trypsin-like protease having a sequence identity of at least 70% compared to SEQ ID NO: 1;
- a prosequence located upstream of the trypsin-like protease and having at its C-terminus a basic amino acid residue, preferably K or R, more preferably wherein the prosequence consists of an amino acid sequence having a sequence identity of at least 70% compared to any of SEQ ID NO:7 to SEQ ID NO:37;
- a bacterial secretion signal sequence located upstream of the trypsin-like protease and of the optionally present prosequence, wherein the bacterial secretion signal sequence is capable of directing secretion in a *Bacillus* host cell to a culture medium, preferably wherein the bacterial secretion signal sequence originates from *Bacillus;*
thereby providing a culture medium comprising the mature trypsin-like protease;
(b) optionally, working-up the cell culture obtained in step (a) thereby providing a solid cell culture residue and a liquid cell culture supernatant;
(c) optionally, purifying the mature trypsin-like protease from the cell culture supernatant;
(d) optionally, formulating the mature trypsin-like protease; and
(e) optionally, packaging the purified mature trypsin-like protease.

2. The method according to claim 1, wherein the prosequence has at its N-terminus an aliphatic amino acid residue, preferably A.

3. The method according to any of the preceding claims, wherein the bacterial secretion signal sequence originates from a species within the genus *Bacillus* selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis.*

4. The method according to any of the preceding claims, wherein the bacterial secretion signal sequence is the secretion signal sequence from secretory *Bacillus* proteins selected from the group consisting of YjfA, YfhK, Csn, LytD, Bpr, WapA, BglC/EglS, LytB, LipA/EstA, YckD, YbdN, YobB, YhfM, BglS, YjdB, YbbE, GlpQ, SacC, YurI/Bsn, PhoB, YdjM, AbnA, YwjE/ClsB, YqgA, LipB/EstB, FliZ, DacB, SacB, YrvJ, YlaE, YqxI, NprB, YbfO, YlqB, SpoIID, YwmC, YvbX, YkvV/StoA, XynA, YbbC, YkvT, NprE, YolC, YqzG, YvcE/CwlO, YkwD, YqxM, Mpr, TasA, YwmD, DacF, LytF, YjiA, PbpD, PelB, SpoIIQ, CccA, CitH/mdh, AspB, YlbL, YoqH, YpbG, YpcP/ExoA, YpuD, YvpB, YwcI, YweA, YwgB, YwmB, YwoF, YwsB, YwtF, Pel, PenP, LytC, YfkN, YngK, YwqC, RpmG, YojL/CwlS, YncM, PhrK, Vpr, PhrC, YfkD, YolA, YoaW, YlxY, SleB, YxiT, NucB, YpuA, YwaD, YpjP, WprA, YjcM, MotB, YdhT/GmuG, LytE, PhrF, YlxW, YobV, YocH, YodV/Phy, YoqM, YraJ, YuaB, YusW, YvgO, YvnB, YxiA/AbnB, CwlD, DltD, FliL, LytR, MreC, PbpB, PhoA, SpoIIP, SpoIIR, YrrR/PbpI, YrrS, YveB/LevB, LytH, YbdG, PbpX, YddT, YjcN, YolIBdbA, YqzC, YlxF, YndA, YkoJ, YwtD/PgdS, YdbK, YbbR, YknX, YnzA/TatAC, YpmS, YvgVBdbD, AmyE, YybN, YwfM, YhdC, YxaK/YxaL, YopL, YhjA, PhrG, YvpA/PelC and PhrA; preferably PhoA, YncM, YqgA, YjfA, YfkD, StoA, YdjM, DacF, Vpr, YbfO, AbnA, LytC, PgdS, YckD, YpuA, YuaB, YvnB, YxaL YoqH and YbdN.

5. The method according to any of the preceding claims, wherein the bacterial secretion signal sequence consists of an amino acid sequence having a sequence identity of at least 70% compared to any of SEQ ID NO:39 to SEQ ID NO:206, preferably to any of SEQ ID NO:39 to SEQ ID NO:75, more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52, SEQ ID NO:56, SEQ ID NO:58, SEQ ID NO:61, SEQ ID NO:62, SEQ ID NO:63, SEQ ID NO:64, SEQ ID NO:66, SEQ ID NO:68, SEQ ID NO:68, SEQ ID NO:70 and SEQ ID NO:74, yet more preferably to any of the group consisting of SEQ ID NO:39, SEQ ID NO:40, SEQ ID NO:42, SEQ ID NO:43, SEQ ID NO:44, SEQ ID NO:45, SEQ ID NO:48, SEQ ID NO:50, SEQ ID NO:52 and SEQ ID NO:56.

6. The method according to any of the preceding claims, wherein step (a) involves processing the preproenzyme into a proenzyme upon expression and secretion, such that:
(i) the bacterial secretion signal sequence is cleaved off the preproenzyme during or after secretion from the *Bacillus* host cell; and
(ii) the resulting proenzyme is located in the culture medium and comprises the prosequence and the trypsin-like protease.

7. The method according to any of the preceding claims, wherein step (a) involves autocatalytically activating the proenzyme such that the prosequence is cleaved off from the trypsin-like protease thereby releasing the mature trypsin-like protease having trypsin-like activity.

8. The method according to any of the preceding claims, wherein the autocatalytic activation takes places in the culture medium without rebuffering steps.

9. The method according to any of the preceding claims, wherein the mature trypsin-like protease exhibits
- an increased volumetric activity; and/or
- an increased volumetric activity recoverable;
compared to mature the trypsin-like protease prepared by the method according to claim 1 wherein the prosequence consists of SEQ ID NO:2 (wild-type).

10. The method according to any of the preceding claims, wherein the host cell is selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* preferably *B. amyloliquefaciens, B. brevis, B. cereus, B. licheniformis, B. megaterium, B. pumilus,* and *B. subtilis;* more preferably *B. amyloliquefaciens.*

11. The method according to any of the preceding claims, wherein step (a) involves growing the host cell at a temperature range of about 28°C to about 37°C, preferably at about 30°C and in a medium with a pH range of about 6.0 to about 8.5, preferably with a pH range of about 6.3 to about 7.5.

12. A preproenzyme, preferably as defined in any of claims 1 to 9, comprising or consisting of
- a trypsin-like protease having a sequence identity of at least 70% compared to SEQ ID NO:1;
- preferably, a prosequence located upstream of the trypsin-like protease and having at its C-terminus a basic amino acid residue, more preferably K or R; even more preferably wherein the prosequence consists of an amino acid sequence having a sequence identity of at least 70% compared to any of SEQ ID NO:7 to SEQ ID NO:37, yet more preferably compared to any of SEQ ID NO:7 to SEQ ID NO:26; and
- a bacterial secretion signal sequence located upstream of the prosequence, wherein the bacterial secretion signal sequence is capable directing secretion in a Bacillus host cell, preferably which originates from Bacillus.

13. An expression vector comprising a DNA sequence coding for the preproenzyme according to claim 12.

14. A host cell comprising a DNA sequence coding for the preproenzyme according claim 12.

15. The host cell according to claim 14, wherein the host cell is a bacterium; preferably a gram-positive bacterium; more preferably a species selected from the genus Bacillus; still more preferably selected from the group consisting of *B. amyloliquefaciens, B. paralicheniformis, B. aerius, B. aerophilus B. altitudinis, B. alvei, B. anthracis, B. aquimaris, B. atrophaeus, B. badius, B. boroniphilus, B. butanolivorans, B. circulans, B. coagulans, B. coahuilensis, B. cohnii, B. firmus, B. globisporus, B. horikoshii, B. hwajinpoensis, B. idriensi, B. infantis, B. laterosporus, B. luciferensis, B. mojavensis, B. muralis, B. mycoides, B. pasteurii, B. polymyxa, B. pseudofirmus, B. pseudomycoides, B. safensis, B. selenatarsenatis, B. shackletonii, B. simplex, B. sonorensis, B. sphaericus, B. sporothermodurans, B. stearothermophilus, B. subtilis subsp. spizizenii, B. subtilis subsp. subtilis, B. tequilensis, B. thermoleovorans, B. thuringiensis, B. vallismortis, B. velezensis, B. vietnamensis,* and *B. vireti;* yet more preferably *B. amyloliquefaciens, B. brevis, B. cereus, B. licheniformis, B. megaterium, B. pumilus,* and *B. subtilis;* even more preferably *B. amyloliquefaciens.*
